# EUROPEAN PATENT SPECIFICATION

(11) **EP 1 851 228 B1**
(45) Date of publication and mention of the grant of the patent: **23.03.2011**
(21) Application number: 06701061.1
(22) Date of filing: 23.01.2006
(51) Int. Cl.: C07D 495/04, A61K 31/519, A61P 35/00

(54) **PYRIMIDOTHIOPHENE COMPOUNDS HAVING HSP90 INHIBITORY ACTIVITY**
PYRIMIDOTHIOPHENVERBINDUNGEN MIT HSP90-HEMMENDER WIRKUNG
COMPOSES DE PYRIMIDOTHIOPHENE AYANT UNE ACTIVITE D'INHIBITION DE LA PROTEINE HSP90

(30) Priority: 25.01.2005 GB 0501535
(43) Date of publication of application: 07.11.2007
(73) Proprietor: VERNALIS (R&D) LTD, Winnersh, Wokingham Berkshire RG41 5UA (GB); Cancer Research Technology Limited, London WC2A 3NL (GB); The Institute of Cancer Research, London SW7 3RP (GB)
(72) Inventor: MATTHEWS, Thomas P., Belmont, Sutton, Surrey SM2 5NG (GB); CHEUNG, Kwai, Ming, Belmont, Sutton, Surrey SM2 5NG (GB); COLLINS, Ian, Belmont, Sutton, Surrey SM2 5NG (GB); MCDONALD, Edward, Belmont, Sutton, Surrey SM2 5NG (GB); BROUGH, Paul, Andrew, Winnersh RG41 5UA (GB); DRYSDALE, Martin, James, Winnersh RG41 5UA (GB)
(74) Representative: Walls, Alan James
(86) International application number: PCT/GB2006/000219
(87) International publication number: WO 2006/079789

(56) References cited:
- WO-A-02/36075

## Description

This invention relates to substituted bicyclic thieno[2,3-d]pyrimidine (herein referred to as 'pyrimidothiophene') compounds having HSP90 inhibitory activity, to the use of such compounds in medicine, in relation to diseases which are responsive to inhibition of HSP90 activity such as cancers, and to pharmaceutical compositions containing such compounds.

### Background to the invention

Molecular chaperones maintain the appropriate folding and conformation of proteins and are crucial in regulating the balance between protein synthesis and degradation. They have been shown to be important in regulating many important cellular functions, such as cell proliferation and apoptosis (Jolly and Morimoto, 2000; Smith et al., 1998; Smith, 2001).

### Heat Shock Proteins (Hsps)

Exposure of cells to a number of environmental stresses, including heat shock, alcohols, heavy metals and oxidative stress, results in the cellular accumulation of a number of chaperones, commonly known as heat shock proteins (Hsps). Induction of Hsps protects the cell against the initial stress insult, enhances recovery and leads to maintenance of a stress tolerant state. It has also become clear, however, that certain Hsps may also play a major molecular chaperone role under normal, stress-free conditions by regulating the correct folding, degradation, localization and function of a growing list of important cellular proteins.

A number of multigene families of Hsps exist, with individual gene products varying in cellular expression, function and localization. They are classified according to molecular weight, e.g., Hsp70, Hsp90, and Hsp27. Several diseases in humans can be acquired as a result of protein misfolding (reviewed in Tytell et al., 2001; Smith et al., 1998). Hence the development of therapies which disrupt the molecular chaperone machinery may prove to be beneficial. In some conditions (e.g., Alzheimer's disease, prion diseases and Huntington's disease), misfolded proteins can cause protein aggregation resulting in neurodegenerative disorders. Also, misfolded proteins may result in loss of wild type protein function, leading to deregulated molecular and physiological functions in the cell.

Hsps have also been implicated in cancer. For example, there is evidence of differential expression of Hsps which may relate to the stage of tumour progression (Martin et al., 2000; Conroy et al., 1996; Kawanishi et al., 1999; Jameel et al., 1992; Hoang et al., 2000; Lebeau et al., 1991). As a result of the involvement of Hsp90 in various critical oncogenic pathways and the discovery that certain natural products with anticancer activity are targeting this molecular chaperone suggests that inhibiting the function of Hsp90 may be useful in the treatment of cancer. To this end, the first in class natural product 17AAG is currently in Phase II clinical trials.

### Hsp90

Hsp90 constitutes about 1-2% of total cellular protein. In cells, it forms dynamic multi-protein complexes with a wide variety of accessory proteins (referred to as co-chaperones) which appear responsible for regulating the chaperone function. It is essential for cell viability and it exhibits dual chaperone functions (Young et al., 2001). When cells undergo various environmental cellular stresses, Hsp90 forms a core component of the cellular stress response by interacting with many proteins after their native conformation has been altered. Environmental stresses, such as heat shock, heavy metals or alcohol, generate localised protein unfolding. Hsp90 (in concert with other chaperones) binds these unfolded proteins allowing adequate refolding and preventing non-specific aggregation (Smith et al., 1998). In addition, recent results suggest that Hsp90 may also play a role in buffering against the effects of mutation, presumably by correcting the inappropriate folding of mutant proteins (Rutherford and Lindquist, 1998). However, Hsp90 also has an important regulatory role. Under normal physiological conditions, together with its endoplasmic reticulum homologue GRP94, Hsp90 plays a housekeeping role in the cell, maintaining the conformational stability and maturation of many client proteins. These can be subdivided into three groups: (a) steroid hormone receptors (e.g. estrogen receptor, progesterone receptor) (b) Ser/Thr or tyrosine kinases (e.g. Her2, Raf-1, CDK4, and Lck), and (c) a collection of apparently unrelated proteins, e.g. mutant p53 and the catalytic subunit of telomerase hTERT. It has also been shown recently that Hsp90 is responsible for stabilising and activating mutated kinases where the wild type kinase is not an Hsp90 client (for an example see the B-Raf story published in da Rocha Dias et al., 2005). All of these proteins play key regulatory roles in many physiological and biochemical processes in the cell. New client proteins of Hsp90 are being constantly identified; see http://www.picard.ch/downloads/Hsp90interactors.pdf for the most up to date list.

The highly conserved Hsp90 family in humans consists of four genes, namely the cytosolic Hsp90α and Hsp90β isoforms (Hickey et al., 1989), GRP94 in the endoplasmic reticulum (Argon et al., 1999) and Hsp75/TRAP1 in the mitochondrial matrix (Felts et al., 2000). Apart from the differences in sub-cellular localisation, very little is known about the differences in function between Hsp90α/β, GRP94 and TRAP1. Initial reports suggesting that certain client proteins were chaperoned by a specific Hsp90 (e.g. Her2 by Grp94 alone) appear to have been erroneous.

Hsp90 participates in a series of complex interactions with a range of client and regulatory proteins (Smith, 2001). Although the precise molecular details remain to be elucidated, biochemical and X-ray crystallographic studies (Prodromou et al., 1997; Stebbins et al., 1997) carried out over the last few years have provided increasingly detailed insights into the chaperone function of Hsp90.

Following earlier controversy on this issue, it is now clear that Hsp90 is an ATP-dependent molecular chaperone (Prodromou et al, 1997), with dimerisation of the nucleotide binding domains being essential for ATP hydrolysis, which is in turn essential for chaperone function (Prodromou et al, 2000a). Binding of ATP results in the formation of a toroidal dimer structure in which the N terminal domains are brought into closer contact with each other resulting in a conformational switch known as the 'clamp mechanism' (Prodromou and Pearl, 2000b). This conformational switching is, in part, regulated by the various co-chaperones associated with Hsp90 (Siligardi et al., 2004).

### Known Hsp90 Inhibitors

The first class of Hsp90 inhibitors to be discovered was the benzoquinone ansamycin class, which includes the compounds herbimycin A and geldanamycin. They were shown to reverse the malignant phenotype of fibroblasts transformed by the v-Src oncogene (Uehara et al., 1985), and subsequently to exhibit potent antitumour activity in both *in vitro* (Schulte et al., 1998) and *in vivo* animal models (Supko et al., 1995).

Immunoprecipitation and affinity matrix studies have shown that the major mechanism of action of geldanamycin involves binding to Hsp90 (Whitesell et al., 1994; Schulte and Neckers, 1998). Moreover, X-ray crystallographic studies have shown that geldanamycin competes at the ATP binding site and inhibits the intrinsic ATPase activity of Hsp90 (Prodromou et al., 1997; Panaretou et al., 1998). This interruption of the chaperone cycle (through blockage of the ATP turnover) causes the loss of the co-chaperone p23 from the complex and the targeting of the client proteins for degradation via the ubiquitin proteasome pathway. 17-Allylamino, 17-demethoxygeldanamycin (17AAG) retains the property of Hsp90 inhibition resulting in client protein depletion and antitumour activity in cell culture and xenograft models (Schulte et al, 1998; Kelland et al, 1999), but has significantly less hepatotoxicity than geldanamycin (Page et al, 1997). Of interest, 17AAG has been shown to be much more active on tumour cells than its affinity for purified Hsp90 would suggest. This has lead to the suggestion that tumour cells (but not non-tumourigenic cells) contain a high-affinity conformation of Hsp90 to which 17AAG binds more tightly, and confers tumour selectivity on Hsp90 inhibitors (Kamal et al., 2003). 17AAG is currently being evaluated in Phase II clinical trials.

Radicicol is a macrocyclic antibiotic shown to reverse the malignant phenotype of v-*Src* and v-*Ha*-*Ras* transformed fibroblasts (Kwon et al, 1992; Zhao et al, 1995). It was shown to degrade a number of signalling proteins as a consequence of Hsp90 inhibition (Schulte et al., 1998). X-ray crystallographic data confirmed that radicicol also binds to the N terminal domain of Hsp90 and inhibits the intrinsic ATPase activity (Roe et al., 1998). Radicicol lacks antitumour activity *in vivo* due to the unstable chemical nature of the compound.

Coumarin antibiotics are known to bind to bacterial DNA gyrase at an ATP binding site homologous to that of the Hsp90. The coumarin, novobiocin, was shown to bind to the carboxy terminus of Hsp90, i.e., at a different site to that occupied by the benzoquinone ansamycins and radicicol which bind at the N-terminus (Marcu et al., 2000b). However, this still resulted in inhibition of Hsp90 function and degradation of a number of Hsp90-chaperoned signalling proteins (Marcu et al., 2000a). Geldanamcyin cannot bind Hsp90 subsequent to novobiocin; this suggests that some interaction between the N and C terminal domains must exist and is consistent with the view that both sites are important for Hsp90 chaperone properties.

A purine-based Hsp90 inhibitor, PU3, has been shown to result in the degradation of signalling molecules, including Her2, and to cause cell cycle arrest and differentiation in breast cancer cells (Chiosis et al., 2001). Recent studies have identified other purine-based compounds with activity against Her2 and activity in cell growth inhibition assays (Dymock et al 2004; Kasibhatla et al 2003; Llauger et al 2005).

Patent publications WO 2004/050087, WO 2004/056782, WO 2004/072051, WO 2004/096212, WO 2005/000300, WO 2005/021552, WO 2005/034950 relate to Hsp90 inhibitors.

### Hsp90 as a Therapeutic Target

Due to its involvement in regulating a number of signalling pathways that are crucially important in driving the phenotype of a tumour, and the discovery that certain bioactive natural products exert their effects via Hsp90 activity, the molecular chaperone Hsp90 is currently being assessed as a new target for anticancer drug development (Neckers et al., 1999).

The predominant mechanism of action of geldanamycin, 17AAG, and radicicol involves binding to Hsp90 at the ATP binding site located in the N-terminal domain of the protein, leading to inhibition of the intrinsic ATPase activity of Hsp90 (Prodromou et al., 1997; Stebbins et al., 1997; Panaretou et al., 1998).

Inhibition of Hsp90 ATPase activity by 17AAG induces the loss of p23 from the chaperone-client protein complex interrupting the chaperone cycle. This leads to the formation of a Hsp90-client protein complex that targets these client proteins for degradation via the ubiquitin proteasome pathway (Neckers et al., 1999; Whitesell & Lindquist, 2005). Treatment with Hsp90 inhibitors leads to selective degradation of important proteins (for example Her2, Akt, estrogen receptor and CDK4) involved in cell proliferation, cell cycle regulation and apoptosis, processes which are fundamentally important in cancer.

The preclinical development of 17AAG as an anticancer agent has been well documented (Sausville et al., 2003) and is currently undergoing Phase II clinical trials. Phase I clinical trials results have been recently published (Banerji et al., 2005; Goetz et al., 2005; Ramanathan et al., 2005 and Grem et al., 2005). Of all these trials, the one conducted by Banerji et al. proved the most positive with a maximum dose of 450mg/m2/week achieved with PD marker responses in the majority of patients and possible antitumour activity in two patients

Inhibition of Hsp90 function has been shown to cause selective degradation of important signalling proteins involved in cell proliferation, cell cycle regulation and apoptosis, processes which are fundamentally important and which are commonly deregulated in cancer (Hostein et al., 2001). An attractive rationale for developing drugs against this target for use in the clinic is that by simultaneously depleting proteins associated with the transformed phenotype, one may obtain a strong antitumour effect and achieve a therapeutic advantage against cancer versus normal cells. These events downstream of Hsp90 inhibition are believed to be responsible for the antitumour activity of Hsp90 inhibitors in cell culture and animal models (Schulte et al., 1998; Kelland et al., 1999).

Recent work has shown that the acetylation status of Hsp90 also plays a role in the control of the chaperone cycle. Inhibition of HDAC6 by either small molecule inhibitors or through siRNA gene targeting interrupts the chaperone cycle. Such treatments cause client protein degradation in a fashion analogous to small molecule ATP site inhibitors (Kovacs et al, 2005; Aoyagi & Archer, 2005).

### Detailed description of the invention

In one broad aspect the present invention provides a compound of formula (I), or a salt, N-oxide, hydrate, or solvate thereof: 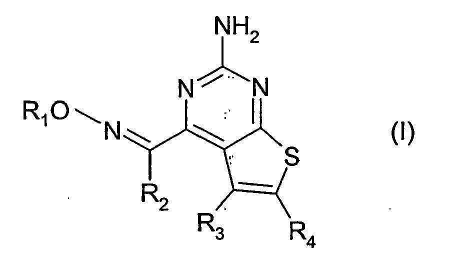 wherein
R₁ is -(Alk¹)ₚ-(Z)ᵣ-(Alk²)ₛ-Q wherein Alk¹ and Alk² are optionally substituted divalent C₁-C₃ alkylene or C₂-C₃ alkenylene radicals,
   p, r and s are independently 0 or 1,
   Z is -O-, -S-, -(C=O)-, -(C=S)-, -SO₂-, -C(=O)O-, -C(=O)NR^{A}- , -C(=S)NR^{A}-, -SO₂NR^{A}-, -NR^{A}C(=O)-, -NR^{A}SO₂- or-NR^{A}-wherein R^{A} is hydrogen or C₁-C₆ alkyl, and
   Q is hydrogen or an optionally substituted carbocyclic or heterocyclic radical;
R₂ is optionally substituted aryl or heteroaryl;
R₃ is hydrogen; and
R₄ is a carboxamide group of formula -CONR^{B}(Alk)ₙR^{A} or a sulphonamide group of formula -SO₂NR^{B}(Alk)ₙR^{A} wherein
   Alk is an optionally substituted divalent alkylene, alkenylene or alkynylene radical,
   n is 0 or 1,
   R^{B} is hydrogen or a C₁-C₆ alkyl or C₂-C₆ alkenyl group,
   R^{A} is hydrogen, C₁-C₆ alkyl or optionally substituted carbocyclic or heterocyclyl,
   or R^{A} and R^{B} taken together with the nitrogen to which they are attached form an N-heterocyclic ring which may optionally contain one or more additional hetero atoms selected from O, S and N, and which may optionally be substituted on one or more ring C or N atoms;
and wherein the terms "substituted" as applied to any moiety , and "aryl" have the meanings defined below.

The invention also concerns the use of such compounds in the preparation of a composition for inhibition of HSP90 activity in vitro or in vivo.

The in vivo use of the invention is applicable to the treatment of diseases in which HSP90 activity is implicated, including use for immunosuppression or the treatment of viral disease, inflammatory diseases such as rheumatoid arthritis, asthma, multiple sclerosis, Type I diabetes, lupus, psoriasis and inflammatory bowel disease; cystic fibrosis angiogenesis-related disease such as diabetic retinopathy, haemangiomas, and endometriosis; or for protection of normal cells against chemotherapy-induced toxicity; or diseases where failure to undergo apoptosis is an underlying factor; or protection from hypoxia-ischemic injury due to elevation of Hsp70 in the heart and brain; scrapie/CJD, Huntingdon's or Alzheimer's disease. Use for the treatment of cancer is especially indicated.

As used herein, the term "(Cₐ-C_{b})alkyl" wherein a and b are integers refers to a straight or branched chain alkyl radical having from a to b carbon atoms. Thus when a is 1 and b is 6, for example, the term includes methyl, ethyl, n-propyl, isopropyl, n-butyl, isobutyl, sec-butyl, t-butyl, n-pentyl and n-hexyl.

As used herein the term "divalent (Cₐ-C_{b})alkylene radical" wherein a and b are integers refers to a saturated hydrocarbon chain having from a to b carbon atoms and two unsatisfied valences.

As used herein the term "(Cₐ-C_{b})alkenyl" wherein a and b are integers refers to a straight or branched chain alkenyl moiety having from a to b carbon atoms having at least one double bond of either E or Z stereochemistry where applicable. The term includes, for example, vinyl, allyl, 1- and 2-butenyl and 2-methyl-2-propenyl.

As used herein the term "divalent (Cₐ-C_{b})alkenylene radical" refers to a hydrocarbon chain having from a to b carbon atoms, at least one double bond, and two unsatisfied valences.

As used herein the term "cycloalkyl" refers to a saturated carbocyclic radical having from 3-8 carbon atoms and includes, for example, cyclopropyl, cyclobutyl, cyclopentyl, cyclohexyl, cycloheptyl and cyclooctyl.

As used herein the term "cycloalkenyl" refers to a carbocyclic radical having from 3-8 carbon atoms containing at least one double bond, and includes, for example, cyclopentenyl, cyclohexenyl, cycloheptenyl and cyclooctenyl.

As used herein the term "aryl" refers to a mono-, bi- or tri-cyclic carbocyclic aromatic radical, and includes aromatic monocyclic or bicyclic carbocyclic radicals fused to a non aromatic carbocyclic or heterocyclic ring. Illustrative of such radicals are phenyl, biphenyl and napthyl, and radicals of the formula: 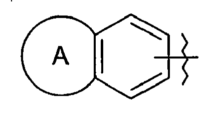 wherein ring A (i) is optionally substituted, (ii) has 5 or 6 ring members including the carbons of the phenyl ring to which it is fused, and (iii) has at least one heteroatom O, S or N hetero atom as a ring member.

As used herein the term "carbocyclic" refers to a cyclic radical whose ring atoms are all carbon, and includes aryl, cycloalkyl, and cycloalkenyl radicals.

As used herein the term "heteroaryl" refers to a mono-, bi- or tri-cyclic aromatic radical containing one or more heteroatoms selected from S, N and O. Illustrative of such radicals are thienyl, benzthienyl, furyl, benzfuryl, pyrrolyl, imidazolyl, benzimidazolyl, thiazolyl, benzthiazolyl, isothiazolyl, benzisothiazolyl, pyrazolyl, oxazolyl, benzoxazolyl, isoxazolyl, benzisoxazolyl, isothiazolyl, triazolyl, benztriazolyl, thiadiazolyl, oxadiazolyl, pyridinyl, pyridazinyl, pyrimidinyl, pyrazinyl, triazinyl, indolyl and indazolyl.

As used herein the unqualified term "heterocyclyl" or "heterocyclic" includes "heteroaryl" as defined above, and in particular refers to a mono-, bi- or tri-cyclic non-aromatic radical containing one or more heteroatoms selected from S, N and O, and to groups consisting of a monocyclic non-aromatic radical containing one or more such heteroatoms which is covalently linked to another such radical or to a monocyclic carbocyclic radical. Illustrative of such radicals are pyrrolyl, furanyl, thienyl, piperidinyl, imidazolyl, oxazolyl, isoxazolyl, thiazolyl, thiadiazolyl, pyrazolyl, pyridinyl, pyrrolidinyl, pyrimidinyl, morpholinyl, piperazinyl, indolyl, morpholinyl, benzfuranyl, pyranyl, isoxazolyl, benzimidazolyl, methylenedioxyphenyl, ethylenedioxyphenyl, maleimido and succinimido groups.

Unless otherwise specified in the context in which it occurs, the term "substituted" as applied to any moiety herein means substituted with at least one substituent, for example selected from (C₁-C₆)alkyl, (C₁-C₆)alkoxy, methylenedioxy, ethylenedioxy, hydroxy, hydroxy(C₁-C₆)alkyl, mercapto, mercapto(C₁-C₆)alkyl, (C₁-C₆)alkylthio, monocyclic carbocyclic of 3-6 ring carbon atoms, monocyclic heterocyclic of 5 or 6 ring atoms, halo (including fluoro and chloro), trifluoromethyl, trifluoromethoxy, nitro, nitrile (-CN), oxo, -COOH, -COOR^{A}, -COR^{A}, -SO₂R^{A}, -CONH₂, -SO₂NH₂, -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A} , -OCONR^{A}R^{B}, -NHCOR^{A}, -NHCOOR^{A}, -NR^{B}COOR^{A}, -NHSO₂OR^{A}, -NR^{B}SO₂OR^{A}, -NHCONH₂, -NR^{A}CONH₂, -NHCONHR^{B}, -NR^{A}CONHR^{B}, -NHCONR^{A}R^{B}, or -NR^{A}CONR^{A}R^{B} wherein R^{A} and R^{B} are independently a (C₁-C₆)alkyl group. In the case where the optional substituent contains an alkyl radical, that alkyl radical may be substituted by a monocyclic carbocyclic group of 3-6 ring carbon atoms, or a monocyclic heterocyclic group of 5 or 6 ring atoms. In the case where the optional substituent is or comprises a monocyclic carbocyclic group of 3-6 ring carbon atoms, or a monocyclic heterocyclic group of 5 or 6 ring atoms, that ring may itself be substituted by any of the non-cyclic optional substituents listed above. An "optional substituent" may be one of the substituent groups encompassed in the above description.

As used herein the term "salt" includes base addition, acid addition and quaternary salts. Compounds of the invention which are acidic can form salts, including pharmaceutically or veterinarily acceptable salts, with bases such as alkali metal hydroxides, e.g. sodium and potassium hydroxides; alkaline earth metal hydroxides e.g. calcium, barium and magnesium hydroxides; with organic bases e.g. N-ethyl piperidine, dibenzylamine and the like. Those compounds (I) which are basic can form salts, including pharmaceutically or veterinarily acceptable salts with inorganic acids, e.g. with hydrohalic acids such as hydrochloric or hydrobromic acids, sulphuric acid, nitric acid or phosphoric acid and the like, and with organic acids e.g. with acetic, tartaric, succinic, fumaric, maleic, malic, salicylic, citric, methanesulphonic and p-toluene sulphonic acids and the like.

For a review on suitable salts, see Handbook of Pharmaceutical Salts: Properties, Selection, and Use by Stahl and Wermuth (Wiley-VCH, Weinheim, Germany, 2002).

The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Compounds with which the invention is concerned which may exist in one or more stereoisomeric form, because of the presence of asymmetric atoms or rotational restrictions, can exist as a number of stereoisomers with R or S stereochemistry at each chiral centre or as atropisomeres with R or S stereochemistry at each chiral axis. The invention includes all such enantiomers and diastereoisomers and mixtures thereof. In particular, the compounds (I) with which the invention is concerned contain an oxime group, the double bond of which may have the cis configuration shown in formula (IA) or the trans configuration shown in formula (IB). Both configurations and mixtures thereof are included within the scope of, and suitable for use in, the invention:

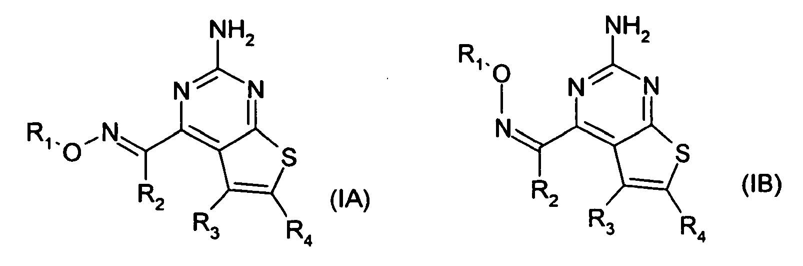

Compounds of the invention may be administered as so-called 'pro-drugs'. Thus certain derivatives of compounds of formula (I) which may have little or no pharmacological activity themselves can, when administered into or onto the body, be converted into compounds of formula (I) having the desired activity, for example, by hydrolytic cleavage. Such derivatives are referred to as 'prodrugs'. Further information on the use of prodrugs may be found in Prodrugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs can, for example, be produced by replacing appropriate functionalities present in the compounds of formula (I) with certain moieties known to those skilled in the art as 'pro-moieties' as described, for example, in Design of Prodrugs by H. Bundgaard (Elsevier, 1985).

Metabolites of compounds of formula (I), that is, compounds formed *in vivo* upon administration of the drug, may be active in their own right as HSP90 inhibitors. Some examples of metabolites include
(i) where the compound of formula (I) contains a methyl group, an hydroxymethyl derivative thereof (-CH₃ -> -CH₂OH):
(ii) where the compound of formula (I) contains an alkoxy group, an hydroxy derivative thereof (-OR -> -OH);
(iii) where the compound of formula (I) contains a tertiary amino group, a secondary amino derivative thereof (-NR¹R² -> -NHR¹ or -NHR²);
(iv) where the compound of formula (I) contains a secondary amino group, a primary derivative thereof (-NHR¹ -> -NH₂);
(v) where the compound of formula (I) contains a phenyl moiety, a phenol derivative thereof (-Ph -> -PhOH); and
(vi) where the compound of formula (I) contains an amide group, a carboxylic acid derivative thereof (-CONH₂ -> COOH).

The compounds of formula (I) defined above and salts, N-oxides hydrates, and solvates thereof are believed novel, and the invention includes all such novel compounds *per se.*

### The radical R₂

R₂ is optionally substituted aryl or heteroaryl, for example phenyl, thienyl, benzthienyl, furyl, benzfuryl, pyrrolyl, imidazolyl, benzimidazolyl, thiazolyl, benzthiazolyl, isothiazolyl, benzisothiazolyl, pyrazolyl, oxazolyl, benzoxazolyl, isoxazolyl, benzisoxazolyl, isothiazolyl, triazolyl, benztriazolyl, thiadiazolyl, oxadiazolyl, pyridinyl, pyridazinyl, pyrimidinyl, pyrazinyl, triazinyl, indolyl or indazolyl. For example, R₂ may be a radical of formula: 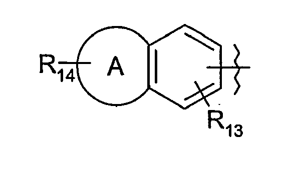 wherein R₁₃and R₁₄ are independently hydrogen or optional substitutents, especially electron donating substituents such as C₁-C₃alkoxy and amino or mono- or di- C₁-C₃alkylamino, or cyclic amino groups, and ring A has 5 or 6 ring members including the carbons of the phenyl ring to which it is fused, and has at least one heteroatom O, S or N hetero atom as a ring member. Examples of the latter type of R₂ group are:

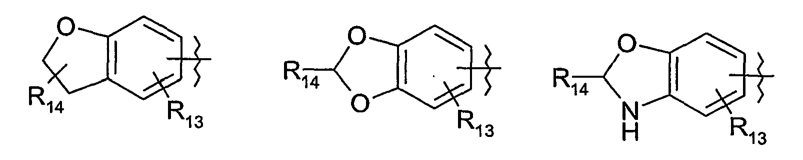

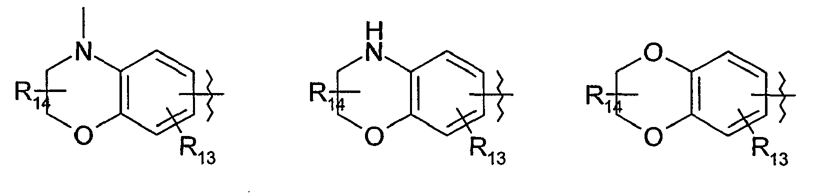

Other specific examples of R₂ radicals are optionally substituted phenyl, naphthyl, 2-, 3-, and 4 pyridyl; 2- and 3-thienyl, 2-, and 3-furyl, 1-, 2- and 3-pyrrolyl, 1-, 2- and 4-imidazolyl, 1, 3- and 4-pyrazolyl. Specific examples of optional substituents which may be present in an R₂ radical, such as a phenyl radical, include chloro, fluoro, methoxy, ethoxy, 1-pyrrolidinyl, and 2-oxopyrrolidin-1-yl of formula

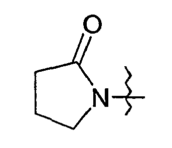

Preferred optional substituents in an an R₂ radical, such as a phenyl radical, are_electron donating substituents such as C₁-C₃alkoxy and amino or mono-or di- C₁-C₃alkylamino, or cyclic amino groups.

### The radical R₁

In a simple embodiment of the compounds with which the invention is concerned, R₁ may be hydrogen or methyl.

In other embodiments R₁ is a radical -(Alk¹)ₚ-(Z)ᵣ-(Alk²)ₛ-Q as defined above. Since the radical R₁ is currently believed not to be involved in any significant interaction with the HSP90 target, it is especially preferred that R₁ be a solubilising radical. In some such embodiments Q is a 5- or 6- membered non-aromatic heterocyclic ring, especially a solubilising ring, such as morpholino, thiomorpholino, piperidinyl, piperazinyl and 2-oxo-pyrrolidinyl. In some cases r is 0 and at least one of p and s is 1, for example p may be 1, s may be 0, and Alk¹ may be -CH₂- or -CH₂CH₂-. In other cases r is 1, p is 1, and s is 0 or 1, for example p may be 1, s may be 0, and Alk¹ may be -CH₂-or -CH₂CH₂-. When r is 1, Z may be, for example -O-, -S-, -NH-, -N(CH₃)-, N(CH₂CH₃)-, -C(=O)NH- , -SO₂NH-, -NHC(=O)-, or -NHSO₂-.

Specific examples of radicals R₁ other than hydrogen and methyl include:

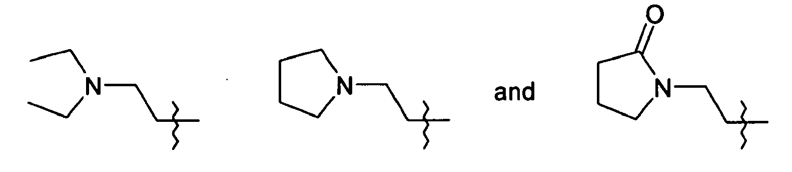

### The optional substituent R₃

R₃ is hydrogen.

### The group R₄

R₄ is a carboxamide group of formula -CONR^{B}(Alk)ₙR^{A} wherein
Alk is a divalent alkylene, alkenylene or alkynylene radical, for example a -CH₂-, -CH(CH₃)-, -CH₂CH₂-, -CH₂CH₂CH₂-, -CH₂CH=CH-, or-CH₂CCCH₂- radical, and the Alk radical may be optionally substituted,
n is 0 or 1,
R^{B} is hydrogen or a C₁-C₆ alkyl or C₂-C₆ alkenyl group, for example methyl, ethyl, n- or iso-propyl, or allyl, Currently it is preferred that R^{B} is hydrogen or a C₁-C₆ alkyl or C₂-C₆ alkenyl group,
R^{A} is hydrogen, or an optional substituent such as hydroxyl; C₁-C₆ alkyl such as methyl or ethyl or C₂-C₆ alkenyl, methoxy; ethoxy; amino (including mono- and di-(C₁-C₃)alkylamino); carbamoyl (-C(=O)NH₂), - SO₂OH; trifluoromethyl; or optionally substituted carbocyclic, for example cyclopropyl, cyclopentyl, cyclohexyl, phenyl optionally substituted by hydroxyl, amino, fluoro, chloro, bromo, 3,4 methylenedioxy, sulfamoyl (-SO₂NH₂) -SO₂OH, methoxy, methylsulfonyl, trifuoromethyl; or heterocyclyl, for example pyridyl, furyl, thienyl, diazolyl, N-piperazinyl, pyrrolyl, tetrahydrofuranyl, thiazolyl, 1-aza-bicyclo[2,2,2]octanyl, or N-morpholinyl any of which heterocyclic rings may be substituted, for example on a ring nitrogen by (C₁-C₃)alkyl. Currently it is preferred that R^{A} is C₁-C₆ alkyl or optionally substituted carbocyclic or heterocyclyl
or R^{A} and R^{B} taken together with the nitrogen to which they are attached form an N-heterocyclic ring which may optionally contain one or more additional hetero atoms selected from O, S and N, and which may optionally be substituted on one or more ring C or N atoms, examples of such N-heterocyclic rings including morpholino, piperidinyl, piperazinyl and N-phenylpiperazinyl. For example R^{A} and R^{B} taken together may be -(CH₂)_{b}-wherein b is 3, 4 or 5, or -CH₂CH₂CH₂CH(=O)-.

Presently it is preferred R₄ groups include ethylamide, iso-propylamide, tert-butylamide, cyclopropylamide, 2-methoxyethylamide, 3-dimethylamino-propylamide, 2-dimethylamino-ethylamide, 2,2,2-trifluoro-ethylamide, and methoxamide.

A specific subgroup of compounds with which the invention is concerned consits of those of formula (I), wherein
(i) in the radical R₁, p r and s are each 0 and Q is hydrogen; or r and s are each 0, p is 1 and Alk¹ is -CH₂- or -CH₂CH₂-, and Q is selected from morpholino, thiomorpholino, piperidinyl, piperazinyl or 2-oxo-pyrrolidinyl;
(ii) R₂ is phenyl, substituted by methylenedioxy, ethylenedioxy, C₁-C₃alkoxy, amino or mono- or di- C₁-C₃alkylamino, or cyclic amino;
(iii) R₃ is hydrogen; and
(iv) R₄ is a carboxamide group of formula -CONR^{B}R^{A} wherein R^{B} is hydrogen or C₁-C₃ alkyl, R^{A} is C₁-C₃ alkyl, or R^{A} and R^{B} taken together are -(CH₂)_{b}- wherein b is 3, 4 or 5, or -CH₂CH₂CH₂CH(=O)-.

Specific compounds with which the invention is concerned include those of the Examples.

There are multiple synthetic strategies for the synthesis of the compounds (I) with which the present invention is concerned, but all rely on known chemistry, known to the synthetic organic chemist. Thus, compounds according to formula (I) can be synthesised according to procedures described in the standard literature and are well-known to the one skilled in the art. Typical literature sources are *"*Advanced organic chemistry", 4th Edition (Wiley), J March, "Comprehensive Organic Transformation", 2nd Edition (Wiley), R.C. Larock, "Handbook of Heterocyclic Chemistry", 2nd Edition (Pergamon), A.R. Katritzky), review articles such as found in *"Synthesis", "Acc. Chem. Res." , "Chem. Rev",* or primary literature sources identified by standard literature searches online or from secondary sources such as *"Chemical Abstracts"* or *"Beilstein".* Such literature methods include those of the preparative Examples herein, and methods analogous thereto.

For example, a ketone of formula (II) 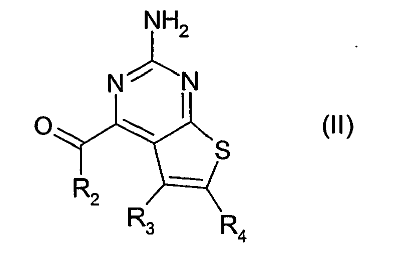 may be reacted with hydroxylamine or a hydroxylamine derivative H₂NOR₁ to form the desired compound of the invention. In general, the desired compound will be formed as a mixture of the cis and trans isomers (I) and (IA), often with a preponderance of one isomer over the other. If a pure isomer is wanted, the isomeric mixture may be separated chromatographically.

Ketones (II) may be prepared by , for example, the following general reaction scheme can be employed:

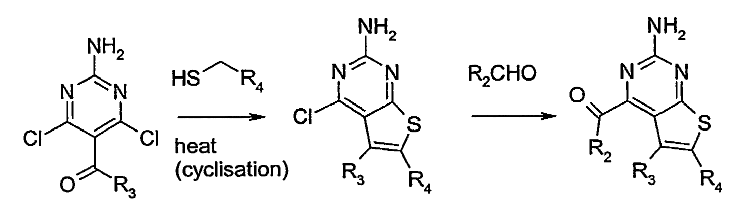

Starting material are either available commercially or can be made according to literature methods. Subsequent reactions may be carried out on R₂, R₃ or R₄ to prepare additional compounds of formula (I).

The compounds of the invention are inhibitors of HSP90 and are useful in the treatment of diseases which are responsive to inhibition of HSP90 activity such as cancers; viral diseases such as Hepatitis C (HCV) (Waxman, 2002); Immunosupression such as in transplantation (Bijlmakers, 2000 and Yorgin, 2000); Anti-inflammatory diseases (Bucci, 2000) such as Rheumatoid arthritis, Asthma, MS, Type I Diabetes, Lupus, Psoriasis and Inflammatory Bowel Disease; Cystic fibrosis (Fuller, 2000); Angiogenesis-related diseases (Hur, 2002 and Kurebayashi, 2001): diabetic retinopathy, haemangiomas, psoriasis, endometriosis and tumour angiogenesis. Also an Hsp90 inhibitor of the invention may protect normal cells against chemotherapy-induced toxicity and be useful in diseases where failure to undergo apoptosis is an underlying factor. Such an Hsp90 inhibitor may also be useful in diseases where the induction of a cell stress or heat shock protein response could be beneficial, for example, protection from hypoxia-ischemic injury due to elevation of Hsp70 in the heart (Hutter, 1996 and Trost, 1998) and brain (Plumier, 1997 and Rajder, 2000). An Hsp90 inhibitor - induced increase in Hsp70 levels could also be useful in diseases where protein misfolding or aggregation is a major causal factor, for example, neurogenerative disorders such as scrapie/CJD, Huntingdon's and Alzheimer's (Sittler, 2001; Trazelt, 1995 and Winklhofer, 2001)".

Ketones of formula (II) above, wherein R₂, R₃ or R₄ are as defined and discussed above in relation to formula (I), are also inhibitors of HSP90 and therefore useful in the same way as the compounds (I) with which the invention is concerned. Such ketones fall within the general class of HSP90 inhibitors disclosed in our copending application PCT/GB2004/003641.

Accordingly, the invention also includes:
(i) A pharmaceutical or veterinary composition comprising a compound of formula (I) above, together with a pharmaceutically or veterinarily acceptable carrier.
(ii) The use of a compound a compound of formula (I) above in the preparation of a composition for composition for inhibition of HSP90 activity in vitro or in vivo.
(iii) A method of treatment of diseases or conditions which are responsive to inhibition of HSP90 activity in mammals which method comprises administering to the mammal an amount of a compound of formula (I) above effective to inhibit said HSP90 activity.

It will be understood that the specific dose level for any particular patient will depend upon a variety of factors including the activity of the specific compound employed, the age, body weight, general health, sex, diet, time of administration, route of administration, rate of excretion, drug combination and the causative mechanism and severity of the particular disease undergoing therapy. In general, a suitable dose for orally administrable formulations will usually be in the range of 0.1 to 3000 mg, once, twice or three times per day, or the equivalent daily amount administered by infusion or other routes. However, optimum dose levels and frequency of dosing will be determined by clinical trials as is conventional in the art.

The compounds with which the invention is concerned may be administered alone, or in combination treatments with other drugs. For example, for the treatment of cancer, the compounds may be administered together with other anticancer drugs, which will normally have a mode of action different from inhibition of HSP90. Treatment of cancer often involves such multi-drug treatments.

The compounds with which the invention is concerned may be prepared for administration by any route consistent with their pharmacokinetic properties. The orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

The active ingredient may also be administered parenterally in a sterile medium. Depending on the vehicle and concentration used, the drug can either be suspended or dissolved in the vehicle. Advantageously, adjuvants such as a local anaesthetic, preservative and buffering agents can be dissolved in the vehicle.

The following examples illustrate the preparation and activities of specific compounds of the invention.

### General Procedures

All reagents obtained from commercial sources were used without further purification. Anhydrous solvents were obtained from commercial sources and used without further drying. Flash chromatography was performed with prepacked silica gel cartridges (Strata SI-1; 61Å, Phenomenex, Cheshire UK or IST Flash II, 54Å, Argonaut, Hengoed, UK). Thin layer chromatography was conducted with 5 x 10 cm plates coated with Merck Type 60 F₂₅₄ silica gel. Ion Exchange Chromatography was performed with IST SCX-2 pre packed cartridges (Argonaut, Hengoed, UK).

The compounds of the present invention were characterized by LC/MS ("method A")using a Hewlett Packard 1100 series LC/MSD linked to quadripole detector (ionization mode: electron spray positive; column: Phenomenex Luna 3u C18(2) 30 x 4.6 mm; Buffer A prepared by dissolving 1.93g ammonium acetate in 2.5 L HPLC grade H₂0 and adding 2 mL formic acid. Buffer B prepared by adding 132 mL buffer A to 2.5 L of HPLC grade acetonitrile and adding 2 mL formic acid; elution gradient 95:5 to 5:95 buffer A : buffer B over 3.5 or 7.5 minutes. Flow rate = 2.0 mL/min).

Some compounds of the invention were characterised by a separate LC/MS system ("method B") using an Agilent 1100 series ion trap XCT (ionisation mode: ESI). Column Genesis 3u C18. Solvent A: 0.1% HCOOH in H₂O;solvent B Acetonitrile. Elution gradient 30:70 to 5:95 solvent A : solvent B over 5 minutes. Flow rate = 1.0 mL/min).

Some compounds of the invention were characterised by a separate LC/MS system ("method C") using a Micromass LCT / Water's Alliance 2795 HPLC system with a Discovery 5 µm, C18, 50 mm x 4.6 mm i.d. column from Supelco at a temperature of 22°C using the following solvent systems: Solvent A: Methanol; Solvent B: 0.1 % Formic acid in water at a flow rate of 1 mL / min. Gradient starting with 10% A / 90% B from 0 - 0.5 mins then 10% A / 90% B to 90% A / 10% B from 0.5 mins to 6.5 mins and continuing at 90% A / 10% B up to 10 mins. From 10-10.5 mins the gradient reverted back to 10% A / 90% where the concentrations remained until 15 mins. UV detection was at 254nm and ionisation was positive or negative ion electrospray. Molecular weight scan range is 50-1000. Samples were supplied as 1mg/mL in DMSO or methanol with 3µL injected on a partial loop fill.

Nuclear magnetic resonance (NMR) analysis was performed with a Brucker DPX-400 MHz NMR spectrometer or a Bruker AV400 400MHz NMR spectrometer The spectral reference was the known chemical shift of the solvent. Proton NMR data is reported as follows: chemical shift (δ) in ppm, integration,multiplicity (s = singlet, d = doublet, t = triplet, q = quartet, p = pentet, m = multiplet, dd = doublet of doublet, br = broad), coupling constant.

Compounds of the invention can be made, by way of example, by the synthetic route shown in scheme 1. Scheme 1 is a general scheme for the synthesis of some compounds of the invention. Suitable reagents, solvents, methodology and reaction temperatures for the synthetic procedures of scheme 1 will be known to those skilled in the art of organic chemistry. Examples of synthetic procedures used to synthesize compound of type E and F are given in examples below.

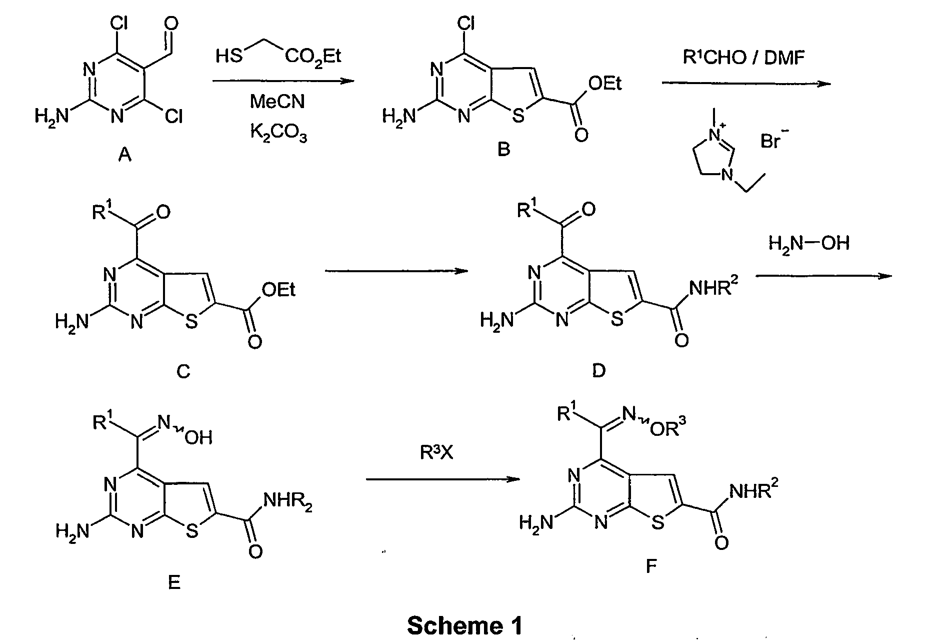

Alternatively, the compounds of type F in scheme 1 can be made by reacting functionalised oximes with ketones of type D. Scheme 2 is a general scheme for this transformation. Examples of synthetic procedures used to synthesize compound of type F by this method are given in example 1.

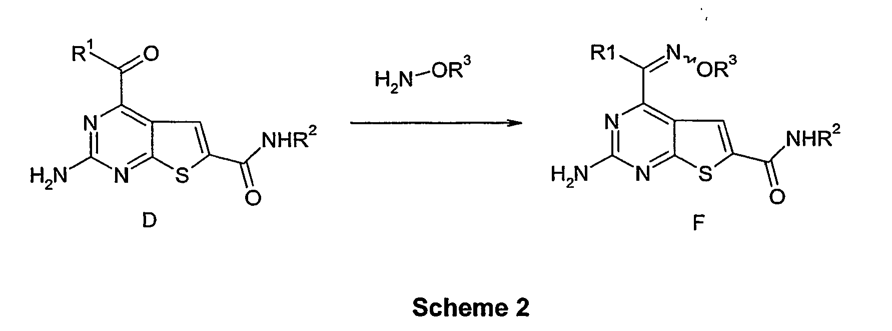

Aldehydes R¹CHO depicted in scheme 1 can be obtained from commercial suppliers or in some cases synthesised by literature methods.

### Example 1

### 2-Amino-4-(methoxyimino-(4-methyl-3,4-dihydro-2H-benzo[1,4]oxazin-7-yl)-methyl]-thieno[2,3-d]pyrimidine-6-carboxylic acid ethylamide

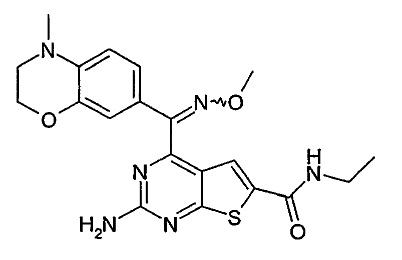

### Step 1

### 2-Amino-4-chloro-thieno[2,3-d]pyrimidine-6-carboxylic acid ethyl ester

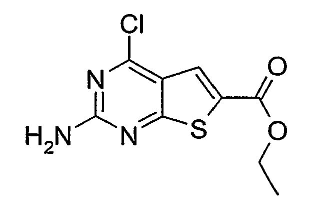

To a stirred mixture of 2-amino-4,6-dichloro-5-formyl-pyrimidine (available from Bionet Research Intermediates, UK) (5.0g 1 eq.) and potassium carbonate (9.0 g; 2.5 equiv.) in acetonitrile (160ml) at ambient temperature was added ethyl-2-mercaptoacetate (2.86 ml; 1.0 equiv.). The resulting mixture was stirred at reflux for three hours. After cooling, the solvents were removed *in vacuo* and the residue partitioned between ethyl acetate and water, the phases separated and the organic phase washed with saturated aqueous sodium chloride solution. Phases were separated and the organic phase was dried over Na₂SO₄ then filtered and filtrate solvents removed *in vacuo.* The crude product was purified by column chromatography on silica gel, eluting with ethyl acetate and hexanes, to afford 2-Amino-4-chloro-thieno[2,3-d]pyrimidine-6-carboxylic acid ethyl ester as a yellow powder (60%).

LC-MS: RT = 2.371 minutes, *m*/*z* = 258.0 [M+H]⁺ (Total run time = 3.5 minutes)

### Step 2

### 2-Amino-4-(4-methyl-3,4-dihydro-2H-benzo[1,4]oxazine-7-carbonyl)-thieno[2,3-d]pyrimidine-6-carboxylic acid ethyl ester

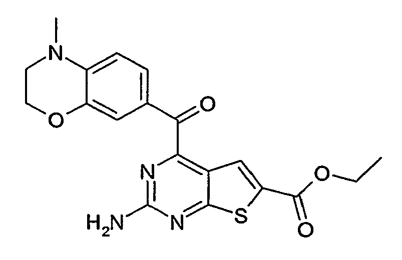

To a solution of 2-amino-4-chloro-thieno[2,3-d]pyrimidine-6-carboxylic acid ethyl ester (0.295 g, 1.14 mmol), 4-methyl-3,4-dihydro-2H-benzo[1,4]oxazine-7-carbaldehyde (0.203 g, 1.14 mmol) and 3-ethyl-1-methyl-3H-imidazol-1-ium bromide (72 mg, 0.38 mmol) in DMF at ambient temperature, was added Sodium Hydride (1.1 mole equiv.). The solution turned dark immediately and was stirred for 3 hours. The resulting orange suspension obtained was filtered through a sinter glass funnel. Aqueous sodium chloride solution was added to the filtrate causing precipitation to occur. The precipitate was then filtered and dried *in vacuo.* The resulting orange solids were purified by preparative TLC. R_{f} = 0.32 (EtOAc : hexane / 3 : 2). This afforded 2-Amino-4-(4-methyl-3,4-dihydro-2H-benzo[1,4]oxazine-7-carbonyl)-thieno[2,3-d]pyrimidine-6-carboxylic acid ethyl ester as an orange powder (100 mg, 20%).

¹H NMR (d₆-acetone) δ = 7.82 (1H, s); 7.55 (1H, dd, J = 8.5 and 2.0 Hz); 7.40 (1H, d, J = 2.0 Hz); 6.75 (2H, s, broad); 6.72 (1H, d, J = 8.5 Hz); 4.30 (2H, q, J = 7.0 Hz); 4.25 (2H, m); 3.50 (2H, m); 3.08 (3H, s) and 1.30 (3H, t, J = 7.0 Hz).
LC/MS:(method C) RT = 6.49 minutes, *m*/*z*= 399.1 (M+H)⁺. (Total run time = 15 minutes)

### Step 3

### 2-Amino-4-(4-methyl-3,4-dihydro-2H-benzo[1,4]oxazine-7-carbonyl)-thieno[2,3-d]pyrimidine-6-carboxylic acid ethylamide

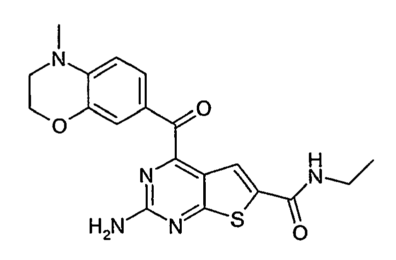

2-Amino-4-(4-methyl-3,4-dihydro-2H-benzo[1,4]oxazine-7-carbonyl)-thieno[2,3-d]pyrimidine-6-carboxylic acid ethyl ester (45 mg, 0.11 mmol) was added to a solution of ethylamine in methanol (15 ml) and the reaction mixture heated and stirred at 85 °C for 4 hours. Methanol was removed *in vacuo* and the residue was dissolved in ethyl acetate and resulting solution then washed with saturated sodium chloride solution. The organic layer was separated and dried over MgSO₄ then filtered and the filtrate solvents removed *in vacuo.* A yellow oil (30 mg, 67%) was obtained after preparative TLC purification (ethyl acetate / hexanes). R_{f} = 0.46 (EtOAc: hexane / 5:1).

¹H NMR (d₆-acetone) δ = 8.00 (1H, s, broad); 7.65 (1H, s); 7.55 (1H, dd, J = 8.7 and 2.0 Hz); 7.38 (1H, d, J = 2.0 Hz); 6.72 (1H, d, J = 8.7 Hz); 6.55 (2H, s, broad); 4.25 (2H, m); 3.50 (2H, m); 3.38 (2H, q, J = 7.0 Hz) 3.05 (3H, s) and 1.25 (3H, t, J = 7.0 Hz).

LC/MS (method C) RT = 6.11 min, *m*/*z* = 398.2 (M+H)⁺. (Total run time = 15 minutes)

### Step 4

### 2-Amino-4-[methoxyimino-(4-methyl-3,4-dihydro-2H-benzo[1,4]oxazin-7-yl)-methyl]-thieno[2,3-d]pyrimidine-6-carboxylic acid ethylamide

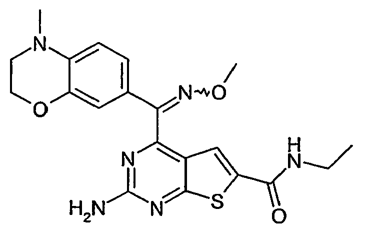

To a solution of 2-Amino-4-(4-methyl-3,4-dihydro-2H-benzo[1,4]oxazine-7-carbonyl)-thieno[2,3-d]pyrimidine-6-carboxylic acid ethylamide (10 mg, 0.025mmol) in ethanol (3 ml) was added methoxylamine hydrochloride (42 mg, 20 equiv.) and the resulting solution irradiated in a microwave reactor at 120 °C for 30 minutes. Ethanol was removed *in vacuo* and the residue partitioned between ethyl acetate and saturated sodium chloride solution. The organic layer was dried over MgSO₄ and evaporated to dryness. Yellow oil (5 mg, 37%) was obtained after preparative TLC purification. Resulting product consists of a mixture of *cis* and *trans* isomers in a ratio of ca. 1:1.7. R_{f} = 0.43 (EtOAc : hexane / 5:2).

(Major isomer): ¹H NMR (d₆-acetone) δ = 7.95 (1H, s, broad); 7.44 (1H, s); 6.91 (1H, d, J = 2.0 Hz); 6.82 (1H, dd, J = 8.5 and 2.0 Hz); 6.63 (1H, d, J = 8.5 Hz); 6.47 (2H, s, broad); 4.25 (2H, m); 3.81 (3H, s); 3.35 (2H + 2H, m); 2.85 (3H, s) and 1.13 (3H, t, J = 7.0 Hz). LC/MS: RT = 6.42 minutes, *m*/*z* = 427.3 (M+H)⁺. (Total run time = 15 minutes)

(Minor isomer): ¹H NMR (d₆-acetone) δ = 7.95 (1H, s, broad); 7.93 (1H, s); 7.05 (1H, d, J = 2.0 Hz); 6.97 (1H, dd, J = 8.5 and 2.0 Hz); 6.65 (1H, d, J = 8.5 Hz); 6.38 (2H, s, broad); 4.25 (2H, m); 4.00 (3H, s); 3.35 (2H + 2H, m); 2.91 (3H, s) and 1.13 (3H, t, J = 7.0 Hz).

LC/MS (method C): RT = 6.63 min, *m*/*z* = 427.3 (M+H)⁺. (Total run time = 15 minutes).

This compound had activity 'A' in the fluorescence polarization assay described below.

### Example 2

### 2-Amino-4-[hydroxyimino-(4-methyl-3,4-dihydro-2H-benzo[1,4]oxazin-7-yl)-methyl]-thieno[2,3-d]pyrimidine-6-carboxylic acid ethylamide

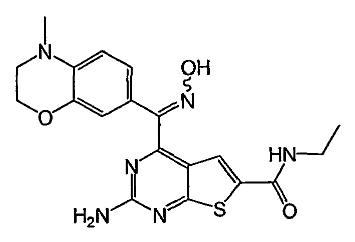

To a solution of 2-Amino-4-(4-methyl-3,4-dihydro-2H-benzo[1,4]oxazine-7-carbonyl)-thieno[2,3-d]pyrimidine-6-carboxylic acid ethylamide (step 3 example 1) (10 mg, 0.025mmol) in ethanol (5 ml) was added hydroxylamine hydrochloride (35 mg, 20 equiv.) and the solution was refluxed for 5 hours. Ethanol was removed *in vacuo* and the residue partitioned between ethyl acetate and brine. The organic layer was dried over MgSO₄ and evaporated to dryness. A yellow oil (4 mg, 39%) was obtained after preparative TLC purification. Resulting product consisted a mixture of cis and trans-isomers in a ratio of ca. 1:1.3. R_{f} = 0.58 (EtOAc : hexane / 5:1).

(Major isomer): ¹H NMR (d₆-acetone) δ = 10.33 (1H, s); 7.98 (1H, s, broad); 7.45 (1H, s); 6.91 (1H, d, J = 2.0 Hz); 6.83 (1H, dd, J = 8.5 and 2.0 Hz); 6.63 (1H, d, J = 8.5 Hz); 6.44 (2H, s, broad); 4.25 (2H, m); 3.81 (3H, s); 3.39 (2H, m); 3.31 (2H, m); 2.90 (3H, s) and 1.10 (3H, t, J = 7.0 Hz). LC/MS (method C): RT = 5.94 min, *m*/*z* = 413.4 (M+H)⁺. (Total run time = 15 minutes)

(Minor isomer): ¹H NMR (d₆-acetone) δ = 10.97 (1H, s); 7.98 (1H, s, broad); 7.91 (1H, s); 7.11 (1H, d, J = 2.0 Hz); 7.03 (1H, dd, J = 8.5 and 2.0 Hz); 6.67 (1H, d, J = 8.5 Hz); 6.35 (2H, s, broad); 4.25 (2H, m); 3.81 (3H, s); 3.39 (2H, m); 3.31 (2H, m); 2.94 (3H, s) and 1.10 (3H, t, J = 7.0 Hz).

LC/MS (method C): RT = 6.09 minutes, m/z 413.4 (M+H)⁺. (Total run time= 15 minutes)

This compound had activity 'A' in the fluorescence polarization assay described below.

### Example 3

### 2-Amino-4-(benzo[1,3]dioxol-5-yl-hydroxyimino-methyl)-thieno[2,3-d]pyrimidine-6-carboxylic acid ethylamide

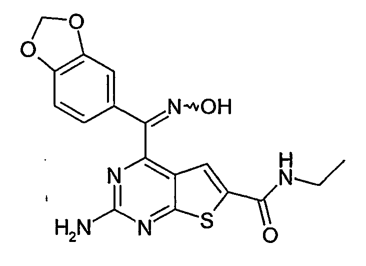

This example was prepared by way of the method of Example 2.

¹H NMR (Acetone-d₆, 250 MHz) (major) δ 10.51 (1H, s), 7.35 (1H, broad s), 7.07 (1H, d, J= 1.1 Hz), 6.69 (2H, d, J= 1.0 Hz), 6.32 (2H, broad s), 5.92 (2H, s), 3.23 (2H, dq, J= 1.6, 7.3 Hz), 1.01 (3H, t, J= 7.2 Hz).
LC/MS (method C) (major) RT = 5.85 min, m/z = 386.2 (M+H)⁺, (minor) RT = 5.99 min, m/z = 386.2 (M+H)⁺. (Total run time = 15 minutes)

This compound had activity 'A' in the fluorescence polarization assay described below.

### Example 4

### 2-Amino-4-(benzo[1,3]dioxol-5-yl-methoxyimino-methyl)-thieno[2,3-d]pyrimidine-6-carboxylic acid ethyl ester

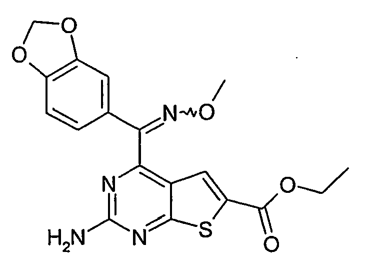

2-Amino-4-(benzo[1,3]dioxole-5-carbonyl)-thieno[2,3-d]pyrimidine-6-carboxylic acid ethyl ester (20 mg, 0.054 mmol) (prepared by way of methods for step 1 and step 2 in Example 1) and methoxylamine hydrochloride (20 equiv.) were dissolved in methanol (1ml) and heated for 10 minutes at 110°C in a CEM microwave reactor (300W, constant cooling). The solvents were removed *in vacuo* and the residue partitioned between water and ethyl acetate. The phases were separated and the organic phase was dried over MgSO₄ and evaporated to dryness *in vacuo.* The residue was purified by preparative TLC eluting with 4% ethanol in dichloromethane to yield the pure product as a yellow powder (2.2 mg, 20%).

(Major isomer) ¹H NMR (CDCl₃, 250 MHz) δ 7.53 (1H, s), 7.13 (1H, d, J= 1.5 Hz), 6.73-6.63 (1H+1H, m), 5.92 (2H, s), 5.31 (2H, broad s), 4.29 (2H, q, J= 7.1 Hz), 3.86 (3H, s), 1.30 (2H, t, J= 7.1 Hz).
LC/MS (method C): RT = 7.85 min, m/z = 401.1 (M+H)⁺. (Total run time = 15 minutes)

(Minor isomer) LC/MS (method C): RT = 7.95 min, *m*/*z* = 401.1 (M+H)⁺. (Total run time = 15 minutes)

This compound had activity 'A' in the fluorescence polarization assay described below.

The following examples (table 1) were synthesised by way of the general synthesis shown in scheme 1 and 2 and by the methods outlined in examples 1 and 2. The column headed "Hsp90 IC50" refers to the activity ('A or B') in the fluorescence polarization assay described below. Retention times are for Method A, 3.5 min run time unless otherwise stated.

**Table 1**

| **Example number** | **Structure** | **LC retention time (method A) mins** | **m/z** | **Hsp90 IC50** |
|---|---|---|---|---|
| 5 | 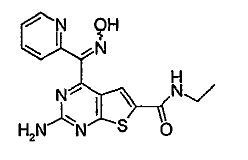 | 1.63 | 343 | A |
| 6 | 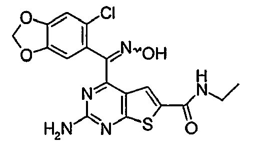 | 2.056 | 420 | A |
| 7 | 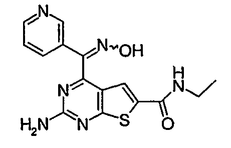 | 1.69 | 343 | B |
| 8 | 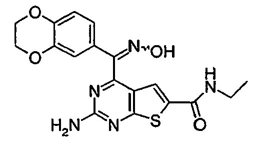 | 2.132 | 385 | B |
| 9 | 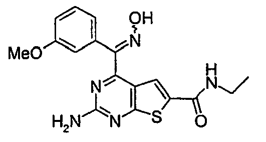 | 2.006 | 372 | A |
| 10 | 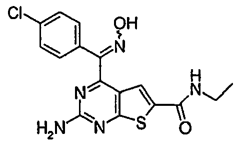 | 2.16 | 376 | A |
| 11 | 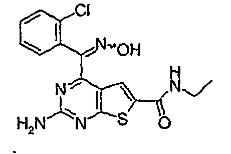 | 2.04 | 376 | B |
| 12 | 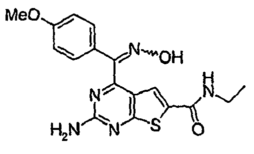 | 1.957 | 372 | A |
| 13 | 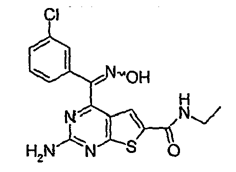 | 2.13 | 376 | A |
| 14 | 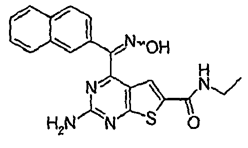 | 2.17 | 392 | A |
| 15 | 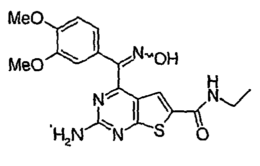 | 1.880 | 402 | A |
| 16 | 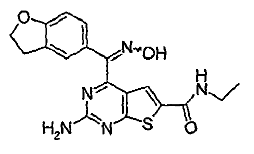 | 1.974 | 384 | A |
| 17 | 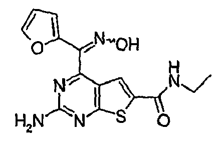 | 1.820 | 332 | A |
| 18 | 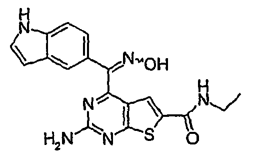 | 1.625 | 381 | A |
| 19 | 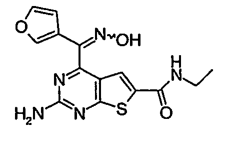 | 1.614 | 332 | A |

### Example 20

### 2-Amino-4-(benzo[1,3]dioxol-5-yl-hydroxyimino-methyl)-thieno[2,3-d]pyrimidine-6-carboxylic acid cyclopropylamide

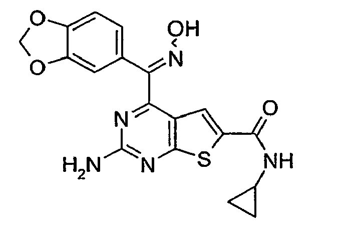

### Step 1

### 2-Amino-4-(benzo[1,3]dioxole-5-carbonyl)-thieno[2,3-d]pyrimidine-6-carboxylic acid ethyl ester

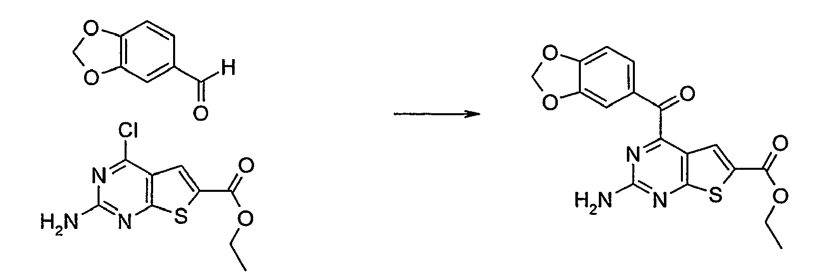

Sodium hydride (0.843g, 21.1 mmol, 60% suspension in oil) was added in one portion to a stirred suspension of 2-amino-4-chloro-thieno[2,3-d]pyrimidine-6-carboxylic acid ethyl ester (4.47g, 17.3 mmol) and piperonal (2.61g, 17.3 mmol) and 3-ethyl-1-methyl-3H-imidazol-1-ium bromide (1.09g, 5.77 mmol) in DMF (54 mL) at room temperature under an atmosphere of nitrogen. The reaction mixture was stirred for 3 hours then poured into a saturated brine solution (300 mL) the resultant emulsion was filtered and the solid dried under vacuum. The solid was purified by flash column chromatography (eluting with 1:2 to 1:1 hexane/ethyl acetate) to give the title compound as a yellow powder (2.57g, 40%). This yellow powder contained ~20% impurities by HPLC.

### Step 2

### 2-Amino-4-(benzo[1,3]dioxol-5-yl-hydroxyimino-methyl)-thieno[2,3-d]pyrimidine-6-carboxylic acid ethyl ester

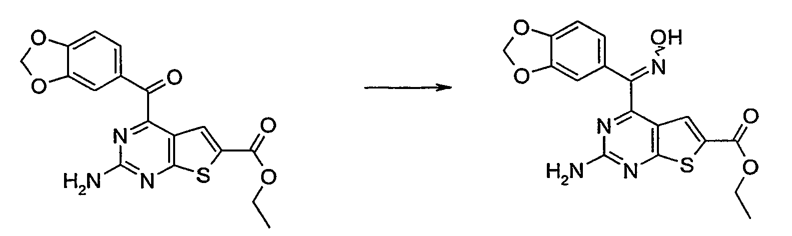

Hydroxylamine hydrochloride (0.973g, 14.0 mmol) was added to a suspension of the ethyl ester (1.30g, ~3.50mmol) in ethanol (50 mL) and the resultant suspension heated at reflux for 4 hours. After this time the reaction mixture was concentrated in *vacuo* to provide a yellow solid which was then partitioned between ethyl acetate (50 mL) and saturated aqueous sodium bicarbonate (50 mL). The organic layer (which contained a significant amount of suspended solid) was then washed with water (50 mL) then saturated brine (50 mL) and then concentrated *in vacuo* to give a highly insoluble yellow solid (1.0g, 74%). [The organic layer was not treated with drying agents or filtered due to the suspended solid]. Attempts to triturate the crude yellow solid with diethyl ether did not improve the purity. The crude solid was used in the next reaction without further purification.

### Step 3

### 2-Amino-4-(benzo[1,3]dioxol-5-yl-hydroxyimino-methyl)-thieno[2,3-d]pyrimidine-6-carboxylic acid

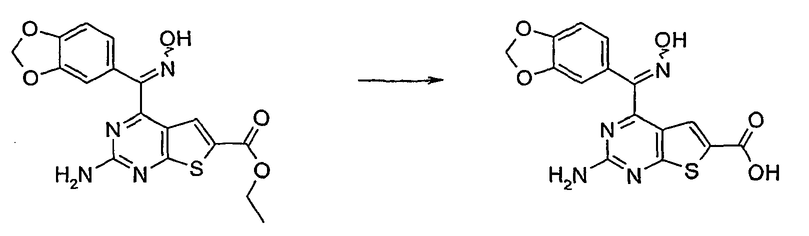

2-Amino-4-(benzo[1,3]dioxol-5-yl-hydroxyimino-methyl)-thieno[2,3-d]pyrimidine-6-carboxylic acid ethyl ester (1.0g, 2.59 mmol) was suspended in a mixture of ethanol (10 mL) and 1.0 M aqueous sodium hydroxide solution (20 mL) and then heated at reflux for one hour after which time the reaction mixture had formed a clear pale yellow solution. The reaction mixture was reduced *in vacuo* to -20 mL then diluted by addition of water (20 mL) then extracted with ethyl acetate (40 mL). The aqueous phase was then acidified by addition of dilute (~ 1 M) aqueous hydrochloric acid then the resultant precipitate was collected by filtration and dried under vacuum at 50 °C for 20 hours to give the title compound as a pale yellow powder (0.890 g, 96%). The product was used without further purification in the next step.

### Step 4

### 2-Amino-4-(benzo[1,3]dioxo)-5-yl-hydroxyimino-methyl)-thieno[2,3-d]pyrimidine-6-carboxylic acid cyclopropylamide

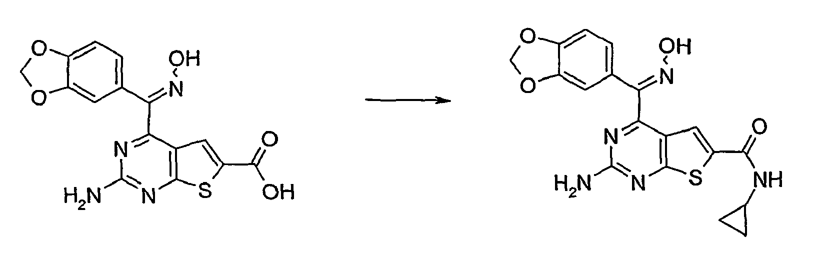

To a solution of the carboxylic acid (40.0 mg, 0.111 mmol), 1-hydroxybenzotriazole (17.0 mg, 0.189 mmol) and cyclopropylamine (5.2 µL, 0.074 mmol) in DMF (2 mL) was added polymer supported carbodiimide resin (123 mg, 0.222 mmol, 1.2 mmol/g loading). The resultant mixture was shaken for 3 hours then polymer supported carbonate resin (236 mg, 1.11 mmol, 3.14 mmol/g loading) was added and the mixture shaken for a further 2 hours. The resin was filtered and washed with dichloromethane (5 mL). The resultant filtrate was then loaded onto an SCX-2 SPE ion exchange column (5g) cartridge. The cartridge was then washed with dichloromethane (15 mL) then methanol (15 mL). The product was then eluted off the SPE cartridge by washing with -7 M solution of ammonia in methanol (15 mL). This latter fraction was concentrated *in vacuo* to give the title compound as a yellow solid (20.0 mg, 68%) in greater than 90% purity by proton NMR.

LC/MS: RT = 2.00 min, *m*/*z* = 398 (M+H)⁺. (Total run time = 3.5 minutes Method A)

This compound had activity 'A' in the fluorescence polarization assay described below.

The following examples (table 2) were synthesised by way of the by the methods outlined in example 20. The column headed "Hsp90 IC50" refers to the activity of the compound ('A or B') in the fluorescence polarization assay described below. Retention times are for Method A, 3.5 minute run time unless otherwise stated.

**Table 2**

| **Example number** | **Structure** | **LC retention time (method A) mins** | **m/z** | **Hsp90 IC50** |
|---|---|---|---|---|
| 21 | 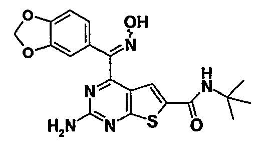 | 2.26 | 414 | A |
| 22 | 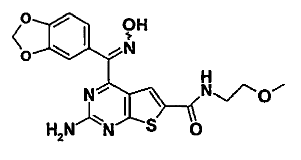 | 1.92 | 416 | A |
| 23 | 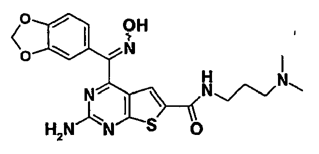 | 1.53 | 443 | A |
| 24 | 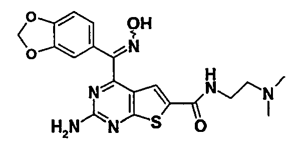 | 1.52 | 429 | A |
| 25 | 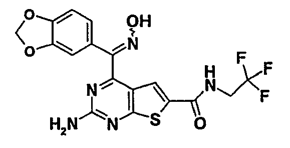 | 2.19 | 440 | A |

### Example 26

### 2-Amino-4-(benzo[1,3]dioxol-5-yl-(2-diethylamino-ethoxyimino)-methyl]-thieno[2,3-d]pyrimidine-6-carboxylic acid ethylamide

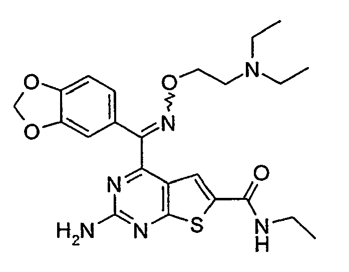

A solution of 2-Amino-4-benzo[1,3]dioxol-5-yl-hydroxyimino-methyl)-thieno[2,3-d]pyrimidine-6-carboxylic acid ethylamide (example 3) (50.0 mg, 130 µmol) and 2-bromo-N,N',diethylamine hydrobromide (37.2 mg, 143 µmol) in DMF (5 mL) was treated with potassium carbonate (90.0 mg, 650 µmol) then heated at 140 °C for 150 minutes. The reaction mixture was purified using an SCX-2 SPE ion exchange cartridge. Purification of the crude product by flash column chromatography (eluting with 1:9:490 cone NH₃(aq):methanol : dichloromethane) gave the product as a pale yellow solid (25.0 mg, 40%). Proton NMR indicated a 3.3:1 ratio of isomers. The major isomer was the less polar isomer (by TLC).
LC/MS: RT = 2.44 and 2.57 min (E/Z regioisomers), *m*/*z* = 485 (M+H)⁺. (Total run time = 7.5 minutes Method A)

This compound had activity 'A' in the fluorescence polarization assay described below.

The following compounds (table 3) were made by way of the general method of scheme 1 utilizing the methods outlined in examples 2, 20 and 27. The column headed "Hsp90 IC50" refers to the activity ('A or B') in the fluorescence polarization assay described below. Retention times are for Method A, 3.5 min run time unless otherwise stated.

**Table 3**

| **Example number** | **Structure** | **LC retention time (method A) mins** | **m/z** | **Hsp90 IC50** |
|---|---|---|---|---|
| 27 | 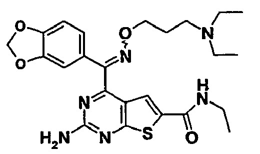 | 1.68 and 1.73 (E/ Z isomers) | 471 | A |
| 28 | 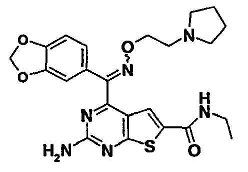 | 2.32 and 2.43 (E/ Z isomers) run time = 7.5 mins | 483 | A |
| 29 | 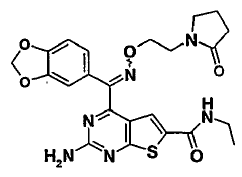 | 3.09 and 3.17 (E/ Z isomers) 7.5 min run time. | 497 | A |
| 30 | 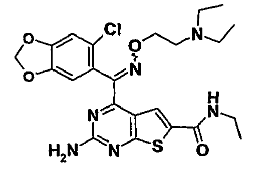 | 2.49 and 2.57 (E/ Z isomers) 7.5 min run time. | 519 | A |
| 31 | 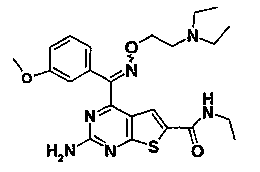 | 1.740 | 471 | A |
| 32 | 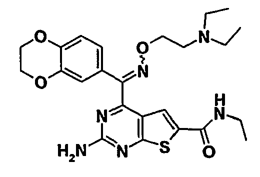 | 1.692 | 499 | A |
| 33 | 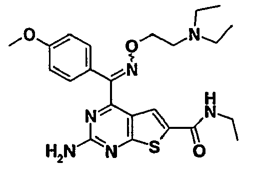 | 1.687 | 471 | A |
| 34 | 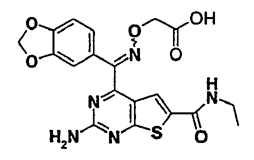 | 1.98 | 444 | A |
| 35 | 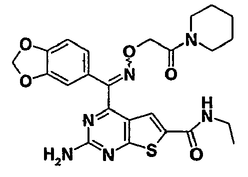 | 2.29 | 511 | A |
| 36 | 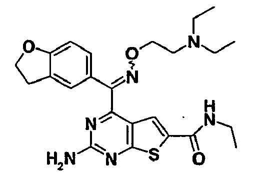 | 1.734 | 483 | A |
| 37 | 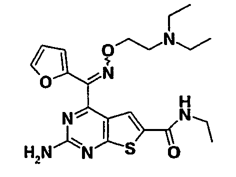 | 1.60 | 431 | B |
| 38 | 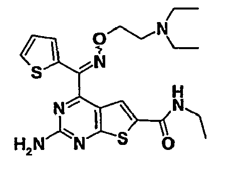 | 0.550 | 447 | B |
| 39 | 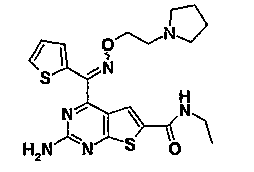 | 0.529 | 445 | B |
| 40 | 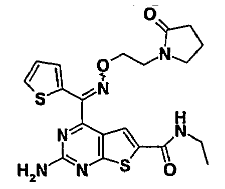 | 0.869 | 459 | A |
| 41 | 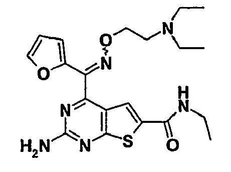 | 0.539 | 431 | A |
| 42 | 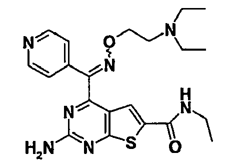 | 0.534 | 442 | B |
| 43 | 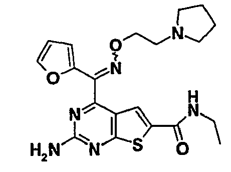 | 0.563 | 429 | A |
| 44 | 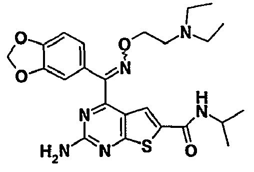 | 0.542 | 499 | A |
| 45 | 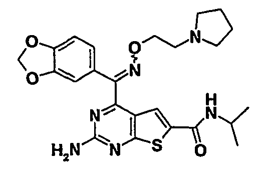 | 0.579 | 497 | A |
| 46 | 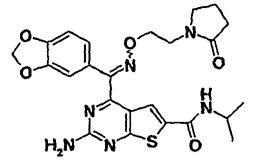 | 0.898; 0.943 (E/ Z isomers) | 511 | A |
| 47 | 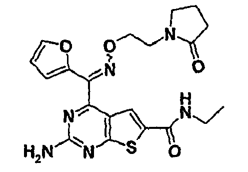 | 0.789 | 443 | A |
| 48 | 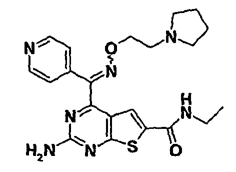 | 0.514 | 440 | B |
| 49 | 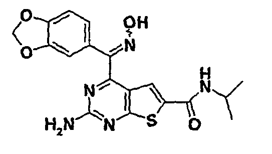 | 0.697 | 454 | A |
| 50 | 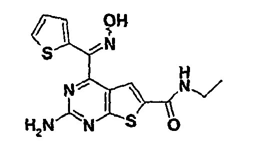 | 0.712 | 348 | A |
| 51 | 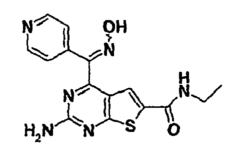 | 0.623 | 343 | B |
| 52 | 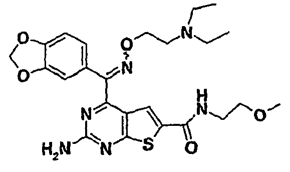 | 0.607 | 515 | A |
| 53 | 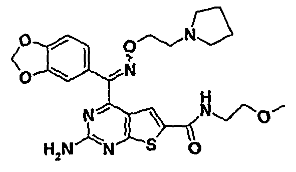 | 0.539 | 513 | A |
| 54 | 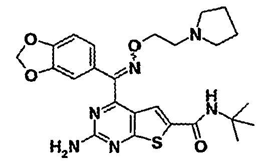 | 0.583; 0.639 (E / Z isomers) | 511 | A |
| 55 | 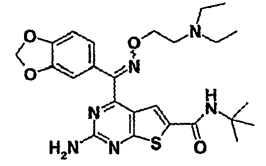 | 0.583 | 513 | A |
| 56 | 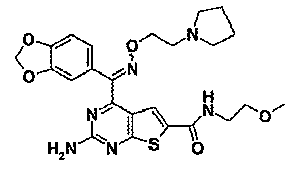 | 0.539 | 513 | A |
| 57 | 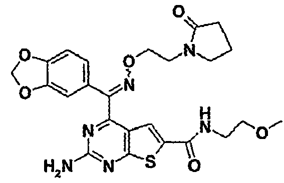 | 0.708; 0.780 (E / Z isomers) | 527 | A |
| 58 | 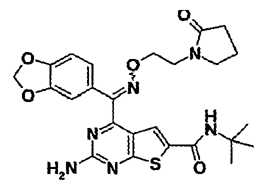 | 1.109 | 525 | A |
| 59 | 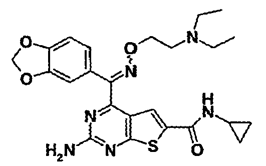 | 0.544 | 497 | A |
| 60 | 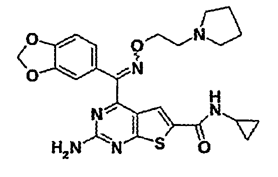 | 0.588 | 495 | A |
| 61 | 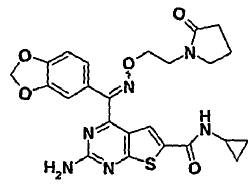 | 0.840 | 509 | A |
| 62 | 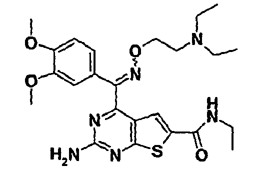 | 1.693 (method A; 3.5 min run time) | 501 | A |
| 63 | 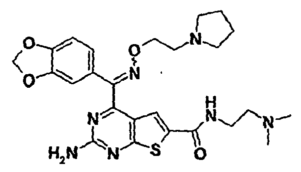 | 0.520 | 526 | B |
| 64 | 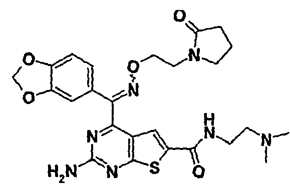 | 0.544 | 540 | B |
| 65 | 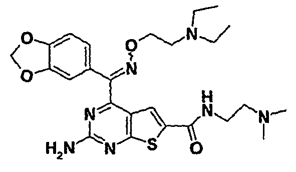 | 0.498 | 528 | B |
| 66 | 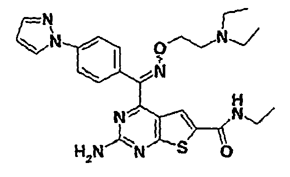 | 0.568 | 501 | A |
| 67 | 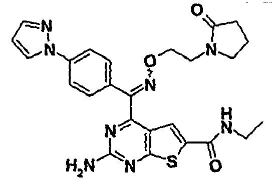 | 0.854 | 519 | A |
| 68 | 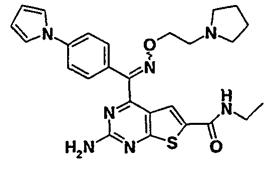 | 0.586 | 504 | B |
| 69 | 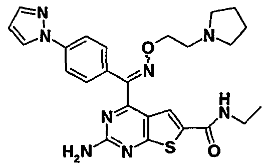 | 0.558 | 505 | B |
| 70 | 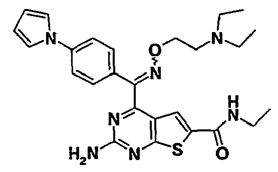 | 0.580 | 506 | B |
| 71 | 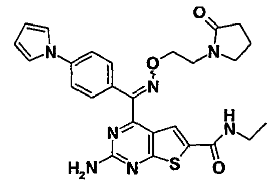 | 1.021; 1.061 | 518 | B |
| 72 | 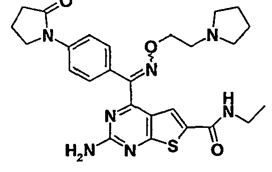 | 0.539 | 522 | A |
| 73 | 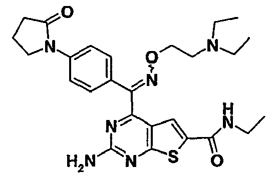 | 0.546 | 524 | B |
| 74 | 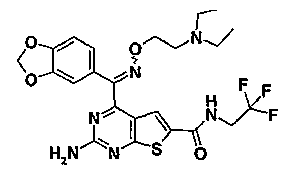 | 0.572 | 539 | A |
| 75 | 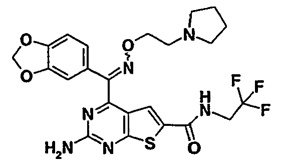 | 0.567 | 537 | A |
| 76 | 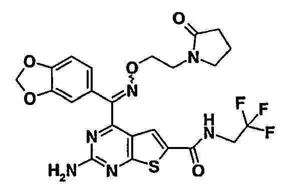 | 0.941;1.018 | 551 | A |
| 77 | 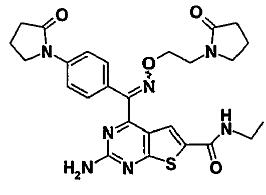 | 0.741 | 536 | A |
| 78 | 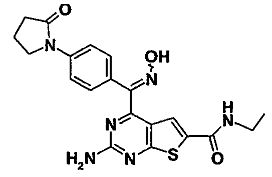 | 0.691 | 425 | A |
| 79 | 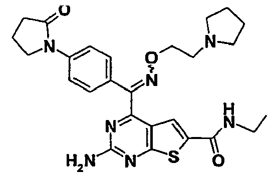 | 0.833 | 440 | A |
| 80 | 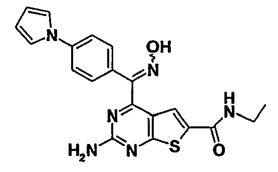 | 0.894 | 407 | A |
| 81 | 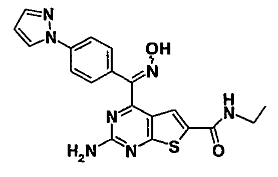 | 0.757 | 408 | A |

### Fluorescence Polarization Assay

Fluorescence polarization {also known as fluorescence anisotropy} measures the rotation of a fluorescing species in solution, where the larger molecule the more polarized the fluorescence emission. When the fluorophore is excited with polarized light, the emitted light is also polarized. The molecular size is proportional to the polarization of the fluorescence emission.

The fluoroscein-labelled probe - 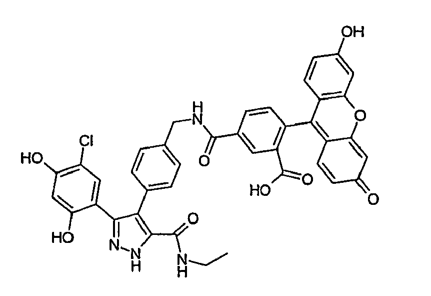 binds to HSP90 { full-length human, full-length yeast or N-terminal domain HSP90 } and the anisotropy {rotation of the probe:protein complex} is measured.

Test compound is added to the assay plate, left to equilibrate and the anisotropy measured again. Any change in anisotropy is due to competitive binding of compound to HSP90, thereby releasing probe.

### Materials

Chemicals are of the highest purity commercially available and all aqueous solutions are made up in AR water.
1) Costar 96-well black assay plate #3915
2) Assay buffer of (a)100mM Tris pH7.4; (b) 20mM KCI; (c) 6mM MgCl₂, Stored at room temperature.
3) BSA (bovine serum albumen) 10 mg/ml (New England Biolabs # B9001S)
4) 20 mM probe in 100 % DMSO stock concentration. Stored in the dark at RT. Working concentration is 200 nM diluted in AR water and stored at 4 °C. Final concentration in assay 80 nM.
5) E. coli expressed human full-length HSP90 protein, purified >95% (see, e.g., Panaretou et al., 1998) and stored in 50µL aliquots at -80°C .
Protocol
1) Add 100µl 1x buffer to wells 11A and 12A (=FP BLNK)
2) Prepare assay mix - all reagents are kept on ice with a lid on the bucket as the probe is light-sensitive.

| | i. Final Concⁿ | |
|---|---|---|
| • 1x Hsp90 FP Buffer | 10 ml | 1x |
| • BSA 10mg/ml (NEB) | 5.0 µl | 5 µg/ml |
| • Probe 200µM | 4.0 µl | 80 nM |
| • Human full-length Hsp90 | 6.25 µl | 200 nM |

3) Aliquot 100µl assay mix to all other wells
4) Seal plate and leave in dark at room temp for 20 minutes to equilibrate Compound Dilution Plate -1 x 3 dilution series
1) In a clear 96-well v-bottom plate - {# VWR 007/008/257} add 10 µl 100% DMSO to wells B1 to H11
2) To wells A1 to A11 add 17.5µl 100% DMSO
3) Add 2.5 µl cpd to A1. This gives 2.5 mM {50x} stock cpd - assuming cpds 20 mM.
4) Repeat for wells A2 to A10. Control in columns 11 and 12.
5) Transfer 5 µl from row A to row B- not column 12. Mix well.
6) Transfer 5 µl from row B to row C. Mix well.
7) Repeat to row G.
8) Do not add any compound to row H - this is the 0 row.
9) This produces a 1x3 dilution series from 50 µM to 0.07 µM.
10)In well B12 prepare 20 µl of 100 µM standard compound.
11)After first incubation the assay plate is read on a Fusion™ α-FP plate reader (Packard BioScience, Pangbourne, Berkshire,UK).
12)After the first read, 2 µl of diluted compound is added to each well for columns 1 to 10. In column 11 {provides standard curve} only add compound B11 - H11. Add 2 µl of 100mM standard cpd to wells B12 - H12 {is positive control }
13)The Z' factor is calculated from zero controls and positive wells. It typically gives a value of 0.7 - 0.9.

The compounds tested in the above assay were assigned to one of two activity ranges, namely A = <10µM; B = >10µM, and those assignments are reported above.

### REFERENCES

A number of publications are cited above in order to more fully describe and disclose the invention and the state of the art to which the invention pertains. Full citations for these references are provided below. Each of these references is incorporated herein by reference in its entirety into the present disclosure.

Reviews for detailed background and drug discovery:
Isaacs JS. 2005 Heat-shock protein 90 inhibitors in antineoplastic therapy: is it all wrapped up?, Expert Opin. Investig. Drugs 14, 569-589.
Maloney A and Workman P. 2002 Hsp90 as a new therapeutic target for cancer therapy: the story unfolds, Expert Opin. Biol. Ther. 2, 3-24.
Whitesell L and Lindquist SL. 2005 Hsp90 and the chaperoning of cancer, Nature Rev. Cancer 5, 761-772.
Janin Y, Heat Shock Protein 90 Inhibitors. A Text Book Example of Medicinal Chemistry? J. Med. Chem. 2005, 48, 7503.
Aoyagi S and Archer TK. 2005 Modulating molecular chaperone Hsp90 functions through reversible acetylation, Trends Cell Biol. 15, 565-7.
Argon Y and Simen BB. 1999 Grp94, an ER chaperone with protein and peptide binding properties, Semin. Cell Dev. Biol. 10, 495-505.
Bijlmakers M-JJE, Marsh M. 2000 Hsp90 is essential for the synthesis and subsequent membrane association, but not the maintenance, of the Src-kinase p561ck, Molecular Biology of the Cell 11, 1585-1595.
Bucci M; Roviezzo F; Cicala C; Sessa WC, Cirino G. 2000 Geldanamycin, an inhibitor of heat shock protein 90 (Hsp90) mediated signal transduction has anti-inflammatory effects and interacts with glucocorticoid receptor in vivo, Brit. J. Pharmacol. 131, 13-16.
Chen C-F, Chen Y, Dai KD, Chen P-L, Riley DJ and Lee W-H. 1996 A new member of the hsp90 family of molecular chaperones interacts with the retinoblastoma protein during mitosis and after heat shock, Mol. Cell. Biol. 16, 4691-4699.
Chiosis G, Timaul MN, Lucas B, Munster PN, Zheng FF, Sepp-Lozenzino L and Rosen N. 2001 A small molecule designed to bind to the adenine nucleotide pocket of Hsp90 causes Her2 degradation and the growth arrest and differentiation of breast cancer cells, Chem. Biol. 8, 289-299.
Conroy SE and Latchman DS. 1996 Do heat shock proteins have a role in breast cancer?, Brit. J. Cancer 74, 717-721.
Dymock B, Barril X, Beswick M, Collier A, Davies N, Drysdale M, Fink A, Fromont C, Hubbard RE, Massey A, Surgenor A, Wright L. Adenine Derived inhibitors of the molecular chaperone HSP90-SAR explained through multiple X-ray structures. Bioorg. Med. Chem. Lett., 2004, 14, 325.
da Rocha Dias S, Friedlos F, Light Y, Springer C, Workman P and Marais R. 2005 Activated B-Raf is an Hsp90 client protein that is targeted by the anticancer drug 17-Allylamino-17-demethoxygeldanamycin, Cancer Res. 65, 10686-10691.
Felts SJ, Owen BAL, Nguyen P, Trepel J, Donner DB and Toft DO. 2000 The Hsp90-related protein TRAP1 is a mitochondrial protein with distinct functional properties, J. Biol. Chem. 5, 3305-3312.
Fuller W, Cuthbert AW. 2000 Post-translational disruption of the delta F508 cystic fibrosis transmembrane conductance regulator (CFTR)-molecular Chaperone complex with geldanamycin stabilizes delta F508 CFTR in the rabbit reticulocyte lysate, J. Biol. Chem. 275(48), 37462-37468.
Hickey E, Brandon SE, Smale G, Lloyd D and Weber LA. 1999 Sequence and regulation of a gene encoding a human 89-kilodalton heat shock protein, Mol. Cell. Biol. 9, 2615-2626.
Hoang AT, Huang J, Rudra-Gonguly N, Zheng J, Powell WC, Rabindron SK, Wu C and Roy-Burman P. 2000 A novel association between the human heat shock transcription factor I (HSF1) and prostate adenocarcinoma, Am. J. Pathol. 156, 857-864.
Hostein I, Robertson D, Di Stefano F, Workman P and Clarke PA. 2001 Inhibition of signal transduction by the Hsp90 inhibitor 17-allylamino-17-demethoxygeldanamycin results in cytostasis and apoptosis, Cancer Res. 61, 4003-4009.
Hur E, Kim H-H, Choi SM, Kim JH, Yim S, Kwon HJ, Choi Y, Kim DK, Lee M-O, Park H. 2002 Reduction of hypoxia-induced transcription through the repression of hypoxia-inducible factor-1α/aryl hydrocarbon receptor nuclear translocator DNA binding by the 90-kDa heat-shock protein inhibitor radicicol, Mol. Pharmacol. 62(5), 975-982.
Hutter et al. 1996 Circulation 94, 1408.
Jameel A, Skilton RA, Campbell TA, Chander SK, Coombes RC and Luqmani YA. 1992 Clinical and biological significance of Hsp89a in human breast cancer, Int. J. Cancer 50, 409-415.
Jolly C and Morimoto RI. 2000 Role of the heat shock response and molecular chaperones in oncogenesis and cell death, J. Natl. Cancer Inst. 92, 1564-1572.
Kamal A, Thao L, Sensintaffar J, Zhang L, Boehm MF, Fritz LC and Burrows FJ. 2003 A high-affinity conformation of Hsp90 confers tumour selectivity on Hsp90 inhibitors, Nature 425, 407-410.
Kasibhatla SR, Hong K, Zhang L, Biamonte MA, Boehm MF, Shi J, Fan J. PCT Int Appl. WO 2003037860.
Kawanishi K, Shiozaki H, Doki Y, Sakita I, Inoue M, Yano M, Tsujinata T, Shamma A and Monden M. 1999 Prognostic significance of heat shock proteins 27 and 70 in patients with squamous cell carcinoma of the esophagus, Cancer 85, 1649-1657.
Kelland LR, Abel G, McKeage MJ, Jones M, Goddard PM, Valenti M, Murrer BA and Harrap KR. 1993 Preclinical antitumour evaluation of bis-acetalo-amino-dichloro-cyclohexylamine platinum (IV): an orally active platinum drug, Cancer Research 53, 2581-2586.
Kelland LR, Sharp SY, Rogers PM, Myers TG and Workman P. 1999 DT-diaphorase expression and tumor cell sensitivity to 17-allylamino, 17-demethoxygeldanamycin, an inhibitor of heat shock protein 90, J. Natl. Cancer Inst. 91, 1940-1949.
Kovacs JJ, Murphy PJM, Gaillard S, Zhao X, Wu J-T, Nicchitta CV, Yoshida M, Toft DO, Pratt WB and Yao T-P. 2005 HDAC6 regulates Hsp90 acetylation and chaperone-dependent activation of the glucocorticoid receptor, Mol. Cell 18, 601-607.
Kurebayashi J, Otsuki T, Kurosumi M, Soga S, Akinaga S, Sonoo, H. 2001 A radicicol derivative, KF58333, inhibits expression of hypoxia-inducible factor-1α and vascular endothelial growth factor, angiogenesis and growth of human breast cancer xenografts, Jap. J. Cancer Res. 92(12), 1342-1351.
Kwon HJ, Yoshida M, Abe K, Horinouchi S and Bepple T. 1992 Radicicol, an agent inducing the reversal of transformed phentoype of src-transformed fibroblasts, Biosci., Biotechnol., Biochem., 56, 538-539.
Lebeau J, Le Cholony C, Prosperi MT and Goubin G. 1991 Constitutive overexpression of 89 kDa heat shock protein gene in the HBL100 mammary cell line converted to a tumorigenic phenotype by the EJ/T24 Harvey-ras oncogene, Oncogene 6,1125-1132.
Llauger L, He, H, Kim J, Aguirre J, Rosen N, Peters U,; Davies P, Chiosis G, J. Med. Chem. 2005, 48, 2892
Marcu MG, Chadli A, Bouhouche I, Catelli M and Neckers L. 2000a The heat shock protein 90 antagonist novobiocin interacts with a previously unrecognized ATP-binding domain in the carboxyl terminus of the chaperone, J. Biol. Chem. 275, 37181-37186.
Marcu MG, Schulte TW and Neckers L. 2000b Novobiocin and related coumarins and depletion of heat shock protein 90-dependent signaling proteins, J. Natl. Cancer Inst. 92, 242-248.
Martin KJ, Kritzman BM, Price LM, Koh B, Kwan CP, Zhang X, MacKay A, O'Hare MJ, Kaelin CM, Mutter GL, Pardee AB and Sager R. 2000 Linking gene expression patterns to therapeutic groups in breast cancer, Cancer Res. 60, 2232-2238.
Neckers L, Schulte TW and Momnaaugh E. 1999 Geldanamycin as a potential anticancer agent: its molecular target and biochemical activity, Invest. New Drugs 17, 361-373.
Page J, Heath J, Fulton R, Yalkowsky E, Tabibi E, Tomaszewski J, Smith A and Rodman L. 1997 Comparison of geldanamycin (NSC-122750) and 17-allylaminogeldanamycin (NSC-330507D) toxicity in rats, Proc. Am. Assoc. Cancer Res. 38, 308.
Panaretou B, Prodromou C, Roe SM, O'Brien R, Ladbury JE, Piper PW and Pearl LH. 1998 ATP binding and hydrolysis are essential to the function of the Hsp90 molecular chaperone in vivo, EMBO J. 17, 4829-4836.
Plumier et al. 1997 Cell. Stress Chap., 2, 162
Pratt WB. 1997 The role of the Hsp90-based chaperone system in signal transduction by nuclear receptors and receptors signalling via MAP kinase, Annu. Rev. Pharmacol. Toxicol. 37, 297-326.
Prodromou C and Pearl LH. 2000a Structure and in vivo function of Hsp90, Curr. Opin. Struct. Biol. 10, 46-51.
Prodromou C, Roe SM, O'Brien R, Ladbury JE, Piper PW and Pearl LH. 1997 Identification and structural characterization of the ATP/ADP-binding site in the Hsp90 molecular chaperone, Cell 90, 65-75.
Prodromou C, Panaretou B, Chohan S, Siligardi G, O'Brien R, Ladbury JE, Roe SM, Piper PW and Pearl LH. 2000b The A TPase cycle of Hsp90 drives a molecular 'clamp' via transient dimerization of the N-terminal domains, EMBO J. 19, 4383-4392.
Rajder et al. 2000 Ann. Neurol. 47, 782*.*
Roe SM, Prodromou C, O'Brien R, Ladbury JE, Piper PW and Pearl LH. 1999 Structural basis for inhibition of the Hsp90 molecular chaperone by the antitumour antibiotics radicicol and geldanamycin, J. Med. Chem. 42, 260-266.
Rutherford SL and Lindquist S. 1998 Hsp90 as a capacitor for morphological evolution. Nature 396, 336-342.
Schulte TW, Akinaga S, Murakata T, Agatsuma T, Sugimoto S, Nakano H, Lee YS, Simen BB, Argon Y, Felts S, Toft DO, Neckers LM and Sharma SV. 1999 Interaction of radicicol with members of the heat shock protein 90 family of molecular chaperones, Mol. Endocrinology 13,1435-1448.
Schulte TW, Akinaga S, Soga S, Sullivan W, Sensgard B, Toft D and Neckers LM. 1998 Antibiotic radicicol binds to the N-terminal domain of Hsp90 and shares important biologic activities with geldanamcyin, Cell Stress and Chaperones 3, 100-108.
Schulte TW and Neckers LM. 1998 The benzoquinone ansamycin 17-allylamino-17-deemthoxygeldanamcyin binds to Hsp90 and shares important biologic activities with geldanamycin, Cancer Chemother. Pharmacol. 42, 273-279.
Siligardi G, Hu B, Panaretou B, Piper PW, Pearl LH and Prodromou C. 2004 Co-chaperone regulation of conformational switching in the Hsp90 ATPase cycle, J. Biol. Chem. 279, 51989-51998.
Sittler et al. 2001 Hum. Mol. Genet. 10, 1307.
Smith DF. 2001 Chaperones in signal transduction, in: Molecular chaperones, in the cell (P Lund, ed.; Oxford University Press, Oxford and NY), 165-178.
Smith DF, Whitesell L and Katsanis E. 1998 Molecular chaperones: Biology and prospects for pharmacological intervention, Pharmacoloctical Reviews 50, 493-513.
Song HY, Dunbar JD, Zhang YX, Guo D and Donner DB. 1995 Identification of a protein with homology to hsp90 that binds the type 1 tumour necrosis factor receptor, J. Biol. Chem. 270, 3574-3581.
Stebbins CE, Russo A, Schneider C, Rosen N, Hartl FU and Pavletich NP. 1997 Crystal structure of an Hsp90-geldanamcyin complex: targeting of a protein chaperone by an antitumor agent, Cell 89, 239-250.
Supko JG, Hickman RL, Grever MR and Malspeis L. 1995 Preclinical pharmacologic evaluation of geldanamycin as an antitumour agent, Cancer Chemother. Pharmacol. 36, 305-315.
Tratzelt et al. 1995 Proc. Nat. Acad. Sci. 92, 2944.
Trost et al. 1998 J. Clin. Invest. 101, 855.
Tytell M and Hooper PL. 2001 Heat shock proteins: new keys to the development of cytoprotective therapies, Emerging Therapeutic Targets 5, 267-287.
Uehara U, Hori M, Takeuchi T and Umezawa H. 1986 Phenotypic change from transformed to normal induced by benzoquinoid ansamycins accompanies inactivation of p60src in rat kidney cells infected with Rous sarcoma virus, Mol. Cell. Biol. 6, 2198-2206.
Waxman, Lloyd H. Inhibiting hepatitis C virus processing and replication. (Merck & Co., Inc., USA). PCT Int. Appl. (2002), WO 0207761
Winklhofer et al. 2001 J. Biol. Chem. 276, 45160.
Whitesell L, Mimnaugh EG, De Costa B, Myers CE and Neckers LM. 1994 Inhibition of heat shock protein Hsp90-pp60v-src heteroprotein complex formation by benzoquinone ansamycins: essential role for stress proteins in oncogenic transformation, Proc. Natl. Acad. Sci. US A. 91, 8324-8328.
Yorgin et al. 2000 Effects of geldanamycin, a heat-shock protein 90-binding agent, on T cell function and T cell nonreceptor protein tyrosine kinases, J. Immunol. 164(6), 2915-2923.
Young JC, Moarefi I and Hartl FU. 2001 Hsp90: a specialized but essential protein-folding tool, J. Cell. Biol. 154, 267-273.
Zhao JF, Nakano H and Sharma S. 1995 Suppression of RAS and MOS transformation by radicicol, Oncogene 11, 161-173.

## Claims

1. A compound of formula (I), or a salt, N-oxide, hydrate, or solvate thereof: 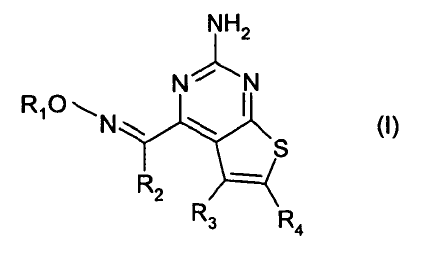 wherein
R₁ is -(Alk¹)ₚ-(Z)ᵣ-(Alk²)ₛ-Q wherein
Alk¹ and Alk² are optionally substituted divalent C₁-C₃ alkylene or C₂-C₃ alkenylene radicals,
p, r and s are independently 0 or 1,
Z is -O-, -S-, -(C=O)-, -(C=S)-, -SO₂-, -C(=O)O-, -C(=O)NR^{A}- ,
- C(=S)NR^{A}-, -SO₂NR^{A}-, -NR^{A}C(=O)-, -NR^{A}SO₂- or -NR^{A}- wherein R^{A} is hydrogen or C₁-C₆ alkyl, and
Q is hydrogen or an optionally substituted carbocyclic or heterocyclic radical;
R₂ is optionally substituted aryl or heteroaryl;
R₃ is hydrogen; and
R₄ is a carboxamide group of formula -CONR^{B}(Alk)ₙR^{A} or a sulphonamide group of formula -SO₂NR^{B}(Alk)ₙR^{A} wherein
Alk is an optionally substituted divalent alkylene, alkenylene or alkynylene radical,
n is 0 or 1,
R^{B} is hydrogen or a C₁-C₆ alkyl or C₂-C₆ alkenyl group,
R^{A} is hydrogen, C₁-C₆ alkyl or optionally substituted carbocyclic or heterocyclyl,
or R^{A} and R^{B} taken together with the nitrogen to which they are attached form an N-heterocyclic ring which may optionally contain one or more additional hetero atoms selected from O, S and N, and which may optionally be substituted on one or more ring C or N atoms;
and wherein the term "substituted" as applied to any moiety means substituted with at least one substituent selected from (C₁-C₆)alkyl, (C₁-C₆)alkoxy, methylenedioxy, ethylentdioxy, hydroxy, hydroxy(C₁-C₆)alkyl, mercapto, mercapto(C₁-C₆)alkyl, (C₁-C₆)alkylthio, monocyclic carbocyclic of 3-6 ring carbon atoms, monocyclic heterocyclic of 5 or 6 ring atoms, halo, trifluoromethyl, trifluoromethoxy, nitro, nitrile (-CN), oxo, -COOH, -COOR^{A}, -COR^{A}, -SO₂R^{A},
- CONH₂, -SO₂NH₂, -CONHR^{A}, -SONHR^{A}, -CONR^{A}R^{B}, -SO₂NR^{A}R^{B}, -NH₂,
- NHR^{A}, -NR^{A}R^{B}, -OCONH₂, -OCONHR^{A}, -OCONR^{A}R^{B}, -NHCOR^{A}, -NHCOOR^{A},
- NR^{B}COOR^{A}, -NHSO₂OR^{A}, -NR^{B}SO₂OR^{A}, -NHCONH₂, -NR^{A}CONH₂,
- NHCONHR^{B}, -NR^{A}CONHR^{B}, -NHCONR^{A}R^{B}, or -NR^{A}CONR^{A}R^{B} wherein R^{A} and R^{B} are independently a (C₁-C₆)alkyl group; and In the case where the optional substituent contains an alkyl radical, that alkyl radical may be substituted by a monocyclic carbocyclic group of 3-6 ring carbon atoms, or a monocyclic heterocyclic group of 5 or 6 ring atoms; and in the case where the optional substituent is or comprises a monocyclic carbocyclic group of 3-6 ring carbon atoms, or a monocyclic heterocyclic group of 5 or 6 ring atoms, that ring may itself be substituted by any of the non-cyclic optional substituents listed above;
and wherein the term "aryl" means a mono-, bi- or tri-cyclic carbocyclic aromatic radical, and includes aromatic monocyclic or bicyclic carbocyclic radicals fused to a non aromatic carbocyclic or heterocyclic ring.

2. A compound as claimed in claim 1 wherein R₂ is optionally substituted phenyl, thienyl, benzthienyl, furyl, benzfuryl, pyrrolyl, imidazolyl, benzimidazolyl, thiazolyl, benzthiazolyl, isothiazolyl, benzisothiazolyl, pyrazolyl, oxazolyl, benzoxazolyl, isoxazolyl, benzisoxazolyl, isothiazolyl, triazolyl, benztriazolyl, thiadiazolyl, oxadiazolyl, pyridinyl, pyridazinyl, pyrimidinyl, pyrazinyl, triazinyl, indolyl or indazolyl.

3. A compound as claimed in claim 1 wherein R₂ is a bicyclic group of formula: 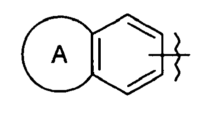 wherein ring A (i) is optionally substituted, (ii) has 5 or 6 ring members including the carbons of the phenyl ring to which it is fused, and (iii) has at least one heteroatom O, S or N hetero atom as a ring member.

4. A compound as claimed in claim 3 wherein R₂ is a radical selected from formulae: 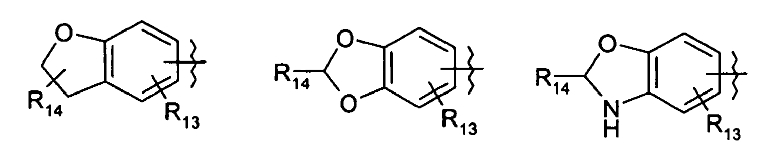 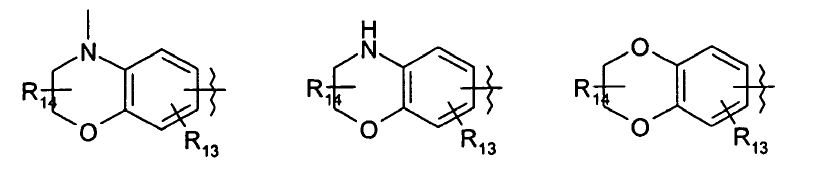 wherein R₁₃ and R₁₄ are independently hydrogen or electron donating substituents,

5. A compound as claimed in claim 4 wherein R₁₃ and R₁₄ are independently hydrogen or C₁-C₃alkoxy, amino or mono- or di- C₁-C₃alkylamino, or cyclic amino groups.

6. A compound as claimed in claim 1 wherein R₂ is optionally substituted phenyl, naphthyl, 2-, 3-, and 4 pyridyl; 2- and 3-thienyl, 2-, and 3-furyl, 1-, 2- and 3-pyrrolyl, 1-, 2- and 4-imidazolyl, 1, 3- and 4-pyrazolyl.

7. A compound as claimed in claim 6 wherein optional substituents which may be present in R₂ are selected from chloro, fluoro, methoxy, ethoxy, 1-pyrrolidinyl, and 2-oxo-pyrrolidin-1-yl:

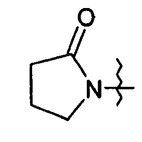

8. A compound as claimed in claim 6 wherein optional substituents which may be present in R₂ are electron donating substituents selected from C₁-C₃alkoxy and amino or mono- or di- C₁-C₃alkylamino, or cyclic amino groups.

9. A compound as claimed in any of the preceding claims wherein R₁ is hydrogen, methyl, ethyl or a radical selected from:

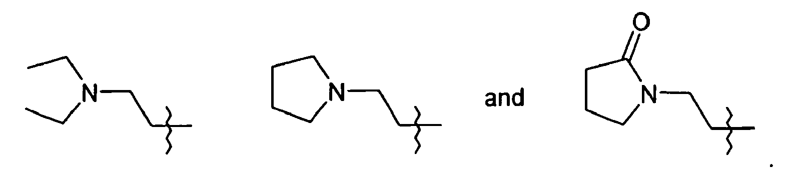

10. A compound as claimed in any of claims 1 to 8 wherein, in the group R₁, Q is a 5- or 6 membered non-aromatic heterocyclic ring.

11. A compound as claimed in claim 10 wherein Q is selected from morpholino, thiomorpholino, piperidinyl, piperazinyl or 2-oxo-pyrrolidinyl.

12. A compound as claimed in any of claims 1 to 11 wherein, in the group R₁, r is 0 and at least one of p and s is 1.

13. A compound as claimed in any of claims 1 to 11 wherein, in the group R₁, p is 1, s is 0, and Alk¹ is -CH₂- or -CH₂CH₂-.

14. A compound as claimed in any of claims 1 to 11 wherein, in the group R₁, r is 1, p is 1, and s is 0 or 1.

15. A compound as claimed in any of claims 1 to 11, 13 or 14 wherein, in the group R₁, r is 1 and Z is -O-, -S-, -NH-, -N(CH₃)-, N(CH₂CH₃)-,
- C(=O)NH- , -SO₂NH-, -NHC(=O)-, or -NHSO₂

16. A compound as claimed in any of the preceding claims wherein, in the group R₄,
Alk is optionally substituted -CH₂-, -CH(CH₃)-, -CH₂CH₂-,
- CH₂CH₂CH₂-, -CH₂CH=CH-, or -CH₂CCCH₂-,
R^{B} is hydrogen or methyl, ethyl, n- or iso-propyl, or allyl,
R^{A} is hydrogen, methyl, ethyl, n- or iso-propyl, or allyl, or optionally substituted phenyl, 3,4 methylenedioxyphenyl, pyridyl, furyl, thienyl, N-piperazinyl, or N-morpholinyl,
or R^{A} and R^{B} R^{B} is hydrogen or C₁-C₃ alkyl, R^{A} is C₁-C₃ alkyl, or R^{A} and R^{B} taken together are -(CH₂)_{b}- wherein b is 3, 4 or 5, or
- CH₂CH₂CH₂CH(=O)-.

17. A compound as claimed in any of claims 1 to 15 wherein R₄ is a carboxamide group selected from ethylamide, iso-propylamide, tert-butylamide, cyclopropylamide, 2-methoxyethylamide, 3-dimethylamino-propylamide, 2-dimethylamino-ethylamide, 2,2,2-trifluoro-ethylamide, and methoxamide.

18. A compound as claimed in claim 1 wherein,
(i) in the radical R₁, p r and s are each 0 and Q is hydrogen; or r and s are each 0, p is 1 and Alk¹ is -CH₂- or -CH₂CH₂-, and Q is selected from morpholino, thiomorpholino, piperidinyl, piperazinyl or 2-oxo-pyrrolidinyl;
(ii) R₂ is phenyl, substituted by methylenedioxy, ethylenedioxy, C₁-C₃alkoxy, amino or mono- or di- C₁-C₃alkylamino, or cyclic amino;
(iii) R₃ is hydrogen; and
(iv) R₄ is a carboxamide group of formula -CONR^{B}R^{A} wherein R^{B} is hydrogen or C₁-C₃ alkyl, R^{A} is C₁-C₃ alkyl, or R^{A} and R^{B} taken together are
- (CH₂)_{b}- wherein b is 3, 4 or 5.

19. A pharmaceutical or veterinary composition comprising a compound as claimed in any of the preceding claims, together with one or more pharmaceutically or veterinarily acceptable carriers and/or excipients.

20. The use of a compound as claimed in any of claims 1 to 18 in the preparation of a composition for the treatment of viral disease, inflammatory diseases such as rheumatoid arthritis, asthma, multiple sclerosis, Type I diabetes, lupus, psoriasis and inflammatory bowel disease; cystic fibrosis angiogenesis-related disease such as diabetic retinopathy, haemangiomas, and endometriosis; or for protection of normal cells against chemotherapy-induced toxicity; or diseases where failure to undergo apoptosis is an underlying factor; or protection from hypoxia-ischemic injury due to elevation of Hsp90 in the heart and brain; scrapie/CJD, Huntingdon's or Alzheimer's disease.

21. The use of a compound as claimed in any of claims 1 to 18 in the preparation of a composition for the treatment of cancer.

## Patentansprüche

1. Verbindung der Formel (I) oder ein Salz, ein N-Oxid, Hydrat oder Solvat davon: 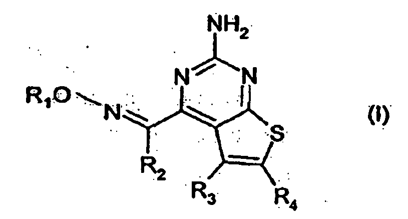 worin R₁ (Alk¹)ₚ-(Z)ᵣ-(Alk²)ₛ-Q ist, worin
Alk¹ und Alk² optional substituierte zweiwertige C₁-C₃-Alkylen- oder C₂-C₃-Alkenylen-Radikale sind,
p, r und s unabhängig voneinander 0 oder 1 sind, Z -O-, -S-, -(C=O)-, -(C=S)-, -SO₂-, -C(=O)O-,
- C(=O)N R^{A}-, -C(=S)N R^{A}-, -SO₂NR^{A}-, -NR^{A}C(=O)-,
- NR^{A}SO₂- oder -NR^{A}- ist, worin R^{A} Wasserstoff oder C₁-C₆-Alkyl ist, und
Q Wasserstoff oder ein optional substituiertes carbocyclisches oder heterocyclisches Radial ist;
R₂ optional substituiertes Aryl oder Heteroaryl ist; R₃ Wasserstoff ist; und
R₄ eine Carboxamidgruppe der Formel -CONR^{B}(Alk)ₙR^{A} oder eine Sulfonamidgruppe der Formel -SO₂NR^{B}(Alk)ₙR^{A} ist, worin
Alk ein optional substituiertes zweiwertiges Alkylen-, Alkenylen- oder Alkinylen-Radikal ist,
n 0 oder 1 ist,
R^{B} Wasserstoff oder eine C₁-C₆-Alkyl- oder C₂-C₆-Alkenylgruppe ist,
R^{A} Wasserstoff, C₁-C₆-Alkyl oder optional substituiertes Carbocyclyl oder Heterocyclyl ist,
oder R^{A} und R^{B} zusammen mit dem Stickstoff, an den sie gebunden sind, einen N-heterocyclischen Ring binden, der optional ein oder mehrere Heteroatome enthalten kann, die aus O, S und N ausgewählt sind, und der optional an einem oder mehreren Ring-C- oder -N-Atomen substituiert sein kann;
und worin der Begriff "substituiert", auf einen beliebigen Rest angewendet, bedeutet: substituiert mit mindestens einem Substituenten, der ausgewählt ist aus (C₁-C₆)-Alkyl, (C₁C₆)-Alkoxy, Methylendioxy, Ethylendioxy, Hydroxy, Hydroxy-(C₁-C₆)-Alkyl, Mercapto, Mercapto-(C₁-C₆)-Alkyl, (C₁-C₆)-Alkylthio, monocyclischer Carbozyklus aus 3 bis 6 Ring-Kohlenstoffatomen, monocyclischer Heterozyklus aus 5 oder 6 Ringatomen, Halo, Trifluoromethyl, Trifluoromethoxy, Nitro, Nitril(-CN), Oxo, -COOH,
- COOR^{A}, -COR^{A}, -SO₂R^{A}, -CONH₂, -SO₂NH₂, -CONHR^{A},
- SO₂NHR^{A}, -CONR^{A}R^{B}, SONR^{A}R^{B}, -NH₂, -NHR^{A}, -NR^{A}R^{B},
- OCONH₂, -OCONHR^{A}, -OCONR^{A}R^{B}, -NHCOR^{A}, -NHCOOR^{A},
- NR^{B}COOR^{A}, -NHSO₂OR^{A}, -NR^{B}SO₂OR^{A}, -NHCONH₂, -NR^{A}CONH₂,
- NHCONHR^{B}, -NR^{A}CONHR^{B}, -NHCONR^{A}R^{B} oder -NR^{A}CONR^{A}R^{B}, worin R^{A} und R^{B} unabhängig voneinander eine (C₁-C₆)-Alkylgruppe sind; und wobei für den Fall, dass der optionale Substituent ein Alkylradikal enthält, das Alkylradikal mit einer monocyclischen carbocyclischen Gruppe aus 3 bis 6 Ring-Kohlenstoffatomen oder einer monocyclischen heterocyclischen Gruppe aus 5 oder 6 Ringatomen substituiert sein kann; und wobei für den Fall, dass der optionale Substituent eine monocyclische carbocyclische Gruppe aus 3 bis 6 Ring-Kohlenstoffatomen oder eine monocyclische heterocyclische Gruppe aus 5 oder 6 Ringatomen ist oder umfasst, der Ring selbst mit beliebigen der oben aufgeführten nicht-cyclischen optionalen Substituenten substituiert sein kann;
und wobei der Begriff "Aryl" ein mono-, bi- oder tricyclisches carbocyclisches aromatisches Radikal bedeutet und aromatische monocyclische oder bicyclische carbocyclische Radikale einschließt, die mit einem nicht-aromatischen carbocyclischen oder heterocyclischen Ring verschmolzen sind.

2. Verbindung gemäß Anspruch 1, worin R₂ optional substituiertes Phenyl, Thienyl, Benzthienyl, Furyl, Benzfuryl, Pyrrolyl, Imidazolyl, Benzimidazolyl, Thiazolyl, Benzthiazolyl, Isothiazolyl, Benzisothiazolyl, Pyrazolyl, Oxazolyl, Benzoxazolyl, Isoxazolyl, Benzisoxazolyl, Isothiazolyl, Triazolyl, Benztriazolyl, Thiadiazolyl, Oxadiazolyl, Pyridinyl, Pyridazinyl, Pyrimidinyl, Pyrazinyl, Triazinyl, Indolyl oder Indazolyl ist.

3. Verbindung gemäß Anspruch 1, worin R₂ eine bicyclische Gruppe der Formel: 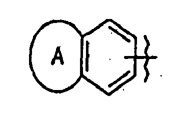 ist, worin der Ring A (i) optional substituiert ist, (ii) 5 oder 6 Rindglieder einschließlich der Kohlenstoffatome des Phenylrings, mit dem er verschmolzen ist, hat, und (iii) mindestens ein Heteroatom O, S oder N als Ringglied hat.

4. Verbindung gemäß Anspruch 3, worin R₂ ein Radikal ist, das aus den Formeln: 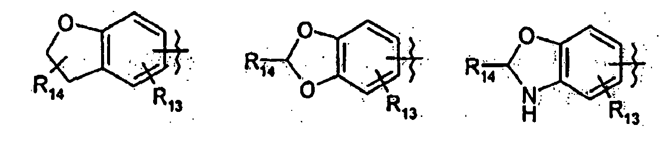 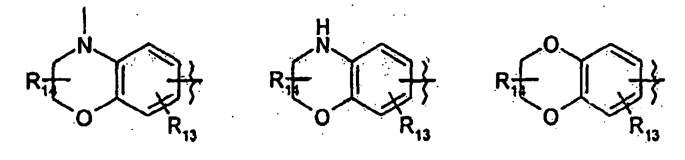 ausgewählt ist, worin R₁₃ und R₁₄ unabhängig voneinander Wasserstoff oder Elektronendonor-Substituenten sind.

5. Verbindung gemäß Anspruch 4, worin R₁₃ und R₁₄ unabhängig voneinander Wasserstoff oder C₁-C₃-Alkoxy, Amino oder Mono- oder Di-C₁-C₃-alkylamino, oder cyclische Aminogruppen sind.

6. Verbindung gemäß Anspruch 1, worin R₂ optional substituiertes Phenyl, Naphthyl, 2-, 3- und 4-Pyridyl; 2- und 3-Thienyl, 2- und 3-Furyl, 1-, 2- und 3-Pyrrolyl, 1-, 2- und 4-Imidazolyl, 1-, 3- und 4-Pyrazolyl ist.

7. Verbindung gemäß Anspruch 6, worin optionale Substituenten, die in R₂ vorhanden sein können, ausgewählt sind aus Chloro, Fluoro, Methoxy, Ethoxy, 1-Pyrrolidinyl und 2-Oxo-pyrrolidin-1-yl:

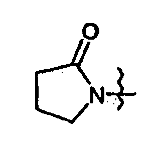

8. Verbindung gemäß Anspruch 6, worin optionale Substituenten, die in R₂ vorhanden sein können, Elektronendonor-Substituenten sind, die aus C₁-C₃-Alkoxy und Amino- oder Mono- oder Di-C₁-C₃-Alkylamino oder cyclischen Aminogruppen ausgewählt sind.

9. Verbindung gemäß mindestens einem der vorhergehenden Ansprüche, worin R₁ Wasserstoff, Methyl, Ethyl oder ein Radikal ist, das ausgewählt ist aus:

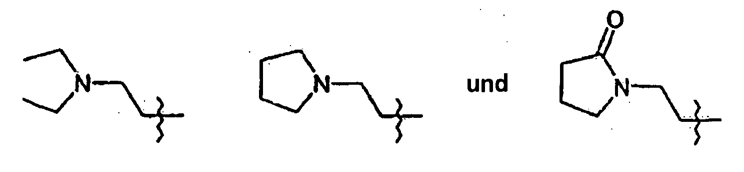

10. Verbindung gemäß mindestens einem der Ansprüche 1 bis 8, worin in der Gruppe R₁ Q ein 5- oder 6-gliedriger nicht-aromatischer heterocyclischer Ring ist.

11. Verbindung gemäß Anspruch 10, worin Q aus Morpholino, Thiomorpholino, Piperidinyl, Piperazinyl oder 2-Oxo-pyrrolidinyl ausgewählt ist.

12. Verbindung gemäß mindestens einem der Ansprüche 1 bis 11, worin in der Gruppe R₁ r gleich 0 ist und mindestens eines von p und s gleich 1 ist.

13. Verbindung gemäß mindestens einem der Ansprüche 1 bis 11, worin in der Gruppe R₁ p gleich 1 ist, s gleich 0 ist und Alk¹ -CH₂- oder -CH₂CH₂- ist.

14. Verbindung gemäß mindestens einem der Ansprüche 1 bis 11, worin in der Gruppe R₁ r gleich 1 ist, p gleich 1 ist und s gleich 0 oder 1 ist.

15. Verbindung gemäß mindestens einem der Ansprüche 1 bis 11, 13 oder 14, worin in der Gruppe R, r gleich 1 ist und Z -O-, -S-, -NH-, -N(CH₃)-, N(CH₂CH₃)-, -C(=O)NH-,
- SO₂NH-, -NHC(=O)- oder -NHSO₂ ist.

16. Verbindung gemäß mindestens einem der vorhergehenden Ansprüche, worin in der Gruppe R₄
Alk optional substituiertes -CH₂-, -CH(CH₃)-, -CH₂CH₂-,
- CH₂CH₂CH₂-, -CH₂CH=CH- oder -CH₂CCCH₂- ist.
R^{B} Wasserstoff oder Methyl, Ethyl, n- oder iso-Propyl oder Allyl ist,
R^{A} Wasserstoff, Methyl, Ethyl, n- oder iso-Propyl oder Allyl oder optional substituiertes Phenyl, 2,4-Methylendioxyphenyl, Pyridyl, Furyl, Thienyl, N-Piperazinyl oder N-Morpholinyl ist,
oder in R^{A} und R^{B} R^{B} Wasserstoff oder C₁-C₃-Alkyl und R^{A} C₁-C₃-Alkyl ist, oder R^{A} und R^{B} zusammengenommen
- (CH₂)_{b}-, mit b gleich 3, 4 oder 5 oder
- CH₂CH₂CH₂CH(=O)- sind.

17. Verbindung gemäß mindestens einem der Ansprüche 1 bis 15, worin R₄ eine Carboxamidgruppe ist, die aus Ethylamid, iso-Propylamid, tert-Butylamid, Cyclopropylamid, 2-Methoxyethylamid, 3-Dimethylaminopropylamid, 2-Dimethlaminoethylamid, 2,2,2-Trifluoroethylamid und Methoxamid ausgewählt ist.

18. Verbindung gemäß Anspruch 1, worin
(i) im Radikal R₁ p, r und s jeweils 0 sind und Q Wasserstoff ist; oder r und s jeweils 0 sind, p gleich 1 ist und Alk¹ -CH₂- oder -CH₂CH₂- ist, und Q aus Morpholino, Thiomorpholino, Piperidinyl, Piperazinyl oder 2-Oxo-pyrrolidinyl ausgewählt ist;
(ii) R₂ Phenyl ist, das mit Methylendioxy, Ethylendioxy, C₁-C₃-Alkoxy, Amino oder Mono-oder Di-C₁-C₃-Alkylamino oder cyclischem Amino substituiert ist;
(iii) R₃ Wasserstoff ist; und
(iv) R₄ eine Carboxyimidgruppe der Formel -CONR^{B}R^{A} ist, worin R^{B} Wasserstoff oder C₁-C₃-Alkyl ist, R^{A} C₁-C₃-Alkyl ist, oder R^{A} und R^{B} zusammengenommen -(CH₂)_{b} mit b gleich 3, 4 oder 5 sind.

19. Pharmazeutische oder veterinär-medizinische Zusammensetzung, die eine Verbindung gemäß mindestens einem der vorhergehenden Ansprüche umfasst, zusammen mit einem oder mehreren pharmazeutisch oder veterinär-medizinisch verträglichen Trägern und/oder Hilfsstoffen.

20. Verwendung der Verbindung gemäß mindestens einem der Ansprüche 1 bis 18 in der Herstellung einer Zusammensetzung zur Behandlung von viraler Erkrankung, Entzündungs-Krankheiten, wie Rheumatoide Arthritis, Asthma, multiple Sklerose, Typ I-Diabetes, Lupus, Psoriasis und entzündlicher Darmerkrankung; zystische Fibrose, Angiogenese-verwandter Krankheiten, wie diabetischer Retinopathie, Hämangiomen und Endometriose; oder zum Schutz normaler Zellen gegen Chemotherapie-induzierte Toxizität; oder Krankheiten, denen das Ausbleiben der Apoptose als Faktor zugrundliegt; oder zum Schutz vor Hypoxie-ischämischer Verletzung aufgrund des Anstiegs von Hsp90 in Herz und Gehirn; Scrapie/CJD, Huntingdon'scher oder Alzheimer'scher Krankheit.

21. Verwendung einer Verbindung gemäß mindestens einem der Ansprüche 1 bis 18 in der Herstellung einer Zusammensetzung zur Behandlung von Krebs.

## Revendications

1. Composé de formule (I), ou sel, N-oxyde, hydrate ou solvate de celui-ci : 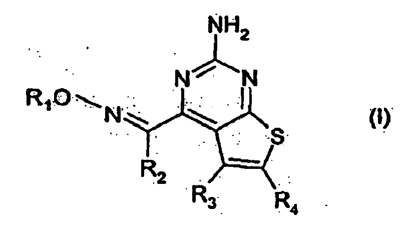 dans lequel
R¹ est -(Alk¹)ₚ-(Z)ᵣ-(Alk²)ₛ-Q, où
Alk¹ et Alk² sont des radicaux alkylène en C₁-C₃ ou alcénylène en C₂-C₃ divalents éventuellement substitués,
p, r et s sont indépendamment 0 ou 1,
Z est -O-, -S-, -(C=O)-, -(C=S)-, -SO₂-, -C(=O)O-, -C(=O)NR^{A}-,
- C(=S)NR^{A}-, -SO₂NR^{A}-, -NR^{A}C(=O)-, -NR^{A}SO₂- ou -NR^{A}-, où R^{A} est l'hydrogène ou un groupe alkyle en C₁-C₆, et
Q est l'hydrogène ou un radical carbocyclique ou hétérocyclique éventuellement substitué ;
R² est un groupe aryle ou hétéroaryle éventuellement substitué ;
R³ est l'hydrogène ; et
R⁴ est un groupe carboxamide de formule -CONR³(Alk)ₙR^{A} ou un groupe sulfonamide de formule -SO₂NR^{B}(Alk)ₙR^{A}, dans lequel
Alk est un radical alkylène, alcénylène ou alcynylène divalent éventuellement substitué,
n vaut 0 ou 1,
R^{B} est l'hydrogène ou un groupe alkyle en C₁-C₆ ou alcényle en C₂-C₆,
R^{A} est l'hydrogène, un groupe alkyle en C₁-C₆ ou un groupe carbocyclique ou hétérocyclyle éventuellement substitué,
ou R^{A} et R^{B} pris ensemble avec l'azote auquel ils sont liés forment un cycle N-hétérocyclique qui peut éventuellement contenir un ou plusieurs hétéroatomes supplémentaires sélectionnés parmi O, S et N, et qui peuvent être éventuellement substitués sur un ou plusieurs atomes C ou N cycliques ;
et dans lequel le terme « substitué », tel qu'appliqué à une fraction quelconque, signifie substitué par au moins un substituant sélectionné parmi des groupes alkyle en C₁-C₆, alcoxy en C₁-C₆, méthylènedioxy, éthylènedioxy, hydroxy, hydroxy-alkyle en C₁-C₆, mercapto, mercapto-alkyle en C₁-C₆, alkyl(en C₁-C₆)-thio, carbocyclique monocyclique de 3 à 6 atomes de carbone cycliques, hétérocyclique monocyclique de 5 ou 6 atomes cycliques, halo, trifluorométhyle, trifluorométhoxy, nitro, nitrile (-CN), oxo, -COOH, -COOR^{A}, -COR^{A}, -SO₂R^{A}, -CONH₂, -SO₂NH₂,
- CONHR^{A}, -SO₂NHR^{A}, -CONR^{A}R^{B}, -SO₂NR^{A}R^{B}, -NH₂, -NHR^{A}, -NR^{A}R^{B},
- OCONH₂, -OCONHR^{A}, -OCONR^{A}R^{B}, NHCOR^{A}, -NHCOOR^{A},
- NR^{B}COOR^{A}, -NHSO₂OR^{A}, -NR^{B}SO₂OR^{A}, -NHCONH₂, -NR^{A}CONH₂,
- NHCONHR^{B}, -NR^{A}CONHR^{B}, -NHCONR^{A}R^{B} ou -NR^{A}CONR^{A}R^{B}, où R^{A} et R^{B} sont indépendamment un groupe alkyle en C₁-C₆ ; et dans le cas où le substituant optionnel contient un radical alkyle, ce radical alkyle peut être substitué par un groupe carbocyclique monocyclique de 3 à 6 atomes de carbone cycliques, ou un groupe hétérocyclique monocyclique de 5 ou 6 atomes cycliques ; et dans le cas où le substituant optionnel est ou comprend un groupe carbocyclique monocyclique de 3 à 6 atomes de carbone cycliques, ou un groupe hétérocyclique monocyclique de 5 ou 6 atomes cycliques, ce cycle peut lui-même être substitué par l'un quelconque des substituants optionnels non cycliques énumérés précédemment ;
et dans lequel le terme « aryle » désigne un radical aromatique carbocyclique mono-, bi- ou tricyclique et comprend des radicaux carbocycliques monocycliques ou bicycliques aromatiques condensés à un cycle carbocyclique ou hétérocyclique non-aromatique.

2. Composé selon la revendication 1, dans lequel R₂ est un groupe, éventuellement substitué, phényle, thiényle, benzothiényle, furyle, benzofuryle, pyrrolyle, imidazolyle, benzimidazolyle, thiazolyle, benzothiazolyle, isothiazolyle, benzisothiazolyle, pyrazolyle, oxazolyle, benzoxazolyle, isoxazolyle, benzisoxazolyle, isothiazolyle, triazolyle, benzotriazolyle, thiadiazolyle, oxadiazolyle, pyridinyle, pyridazinyle, pyrimidinyle, pyrazinyle, triazinyle, indolyle ou indazolyle.

3. Composé selon la revendication 1, dans lequel R₂ est un groupe bicyclique de formule 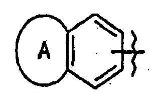 dans laquelle A (i) est éventuellement substitué, (ii) a 5 ou 6 éléments de cycle y compris les carbones du cycle phényle auquel il est condensé, et (iii) a au moins un hétéroatome O, S ou N en tant qu'élément de cycle.

4. Composé selon la revendication 3, dans lequel R₂ est un radical sélectionné parmi les formules : 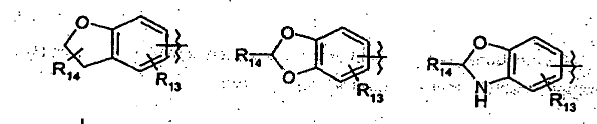 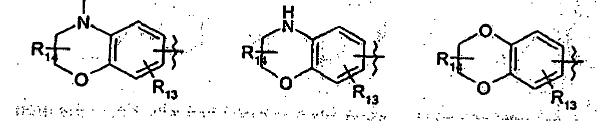 où R₁₃ et R₁₄ sont indépendamment l'hydrogène ou des substituants donneurs d'électron.

5. Composé selon la revendication 4, dans lequel R₁₃ et R₁₄ sont indépendamment l'hydrogène ou des groupes alcoxy en C₁-C₃, amino ou mono- ou di-alkyl(en C₁-C₃)-amino, ou des groupes amino cycliques.

6. Composé selon la revendication 1, dans lequel R₂ est un groupe, éventuellement substitué, phényle, naphtyle, 2-, 3- et 4-pyridyle, 2- et 3-thiényle, 2- et 3-furyle, 1-, 2- et 3-pyrrolyle, 1-, 2- et 4-imidazolyle, 1-, 3- et 4-pyrazolyle.

7. Composé selon la revendication 6, dans lequel les substituants éventuels qui peuvent être présents dans R₂ sont sélectionnés parmi des groupes chloro, fluoro, méthoxy, éthoxy, 1-pyrrolidinyle et 2-oxo-pyrrolidin-1-yle :

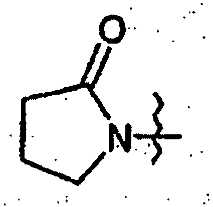

8. Composé selon la revendication 6, dans lequel les substituants éventuels qui peuvent être présents dans R₂ sont des substituants donneurs d'électron sélectionnés parmi des groupes alcoxy en C₁-C₃ et amino ou mono- ou di-alkyl(en C₁-C₃)-amino, ou des groupes amino cycliques.

9. Composé selon l'une quelconque des revendications précédentes, dans lequel R₁ est l'hydrogène, le méthyle, l'éthyle ou un radical sélectionné parmi :

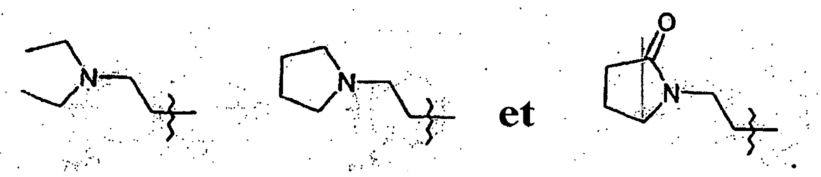

10. Composé selon l'une quelconque des revendications 1 à 8, dans lequel, dans le groupe R₁, Q est un cycle hétérocyclique non-aromatique à 5 ou 6 chaînons.

11. Composé selon la revendication 10, dans lequel Q est sélectionné parmi les groupes morpholino, thiomorpholino, pipéridinyle, pipérazinyle ou 2-oxo-pyrrolidinyle.

12. Composé selon l'une quelconque des revendications 1 à 11, dans lequel, dans le groupe R₁, r vaut 0 et au moins un parmi p et s vaut 1.

13. Composé selon l'une quelconque des revendications 1 à 11, dans lequel, dans le groupe R₁, p vaut 1, s vaut 0 et Alk¹ est -CH₂- ou
- CH₂CH₂-.

14. Composé selon l'une quelconque des revendications 1 à 11, dans lequel, dans le groupe R₁, r vaut 1, p vaut 1 et s vaut 0 ou 1.

15. Composé selon l'une quelconque des revendications 1 à 11, 13 ou 14, dans lequel, dans le groupe R₁, r vaut 1 et Z est -O-, -S-, -NH-,
- N(CH₃)-, -N(CH₂CH₃)-, -C(=O)NH-, -SO₂NH-, -NHC(=O) ou -NHSO₂.

16. Composé selon l'une quelconque des revendications
précédentes, dans lequel, dans le groupe R₄,
Alk est -CH₂-, -CH(CH₃)-, -CH₂CH₂-_{,} -CH₂CH₂CH₂-, -CH₂CH=CH- ou
- CH₂CCCH₂-, éventuellement substitué,
R^{B} est l'hydrogène ou un groupe méthyle, éthyle, n- ou isopropyle ou allyle, R^{A} est l'hydrogène ou un groupe méthyle, éthyle, n- ou isopropyle, ou allyle,
ou un groupe phényle éventuellement substitué, 3-4-méthylènedioxyphényle, pyridyle, furyle, thiényle, N-pipérazinyle ou N-morpholinyle,
ou R^{A} et R^{B}R^{B} forment l'hydrogène ou un groupe alkyle en C₁-C₃, R^{A} est un groupe alkyle en C₁-C₃, ou R^{A} et R^{B} pris conjointement sont -(CH₂)_{b}, où b vaut 3, 4 ou 5, ou -CH₂CH₂CH₂CH(=O)-.

17. Composé selon l'une quelconque des revendications 1 à 15, dans lequel R₄ est un groupe carboxamide sélectionné parmi l'éthylamide, l'iso-propylamide, le tert-butylamide, le cyclopropylamide, le 2-méthoxyéthylamide, le 3-diméthylamino-propylamide, le 2-diméthylamino-éthylamide, le 2,2,2-trifluoro-éthylamide et le méthoxamide.

18. Composé selon la revendication 1, dans lequel
(i) dans le radical R₁, p, r et s valent chacun 0 et Q est l'hydrogène ; ou r et s valent chacun 0, p vaut 1 et Alk¹ est -CH₂- ou -CH₂CH₂-, et Q est sélectionné parmi les groupes morpholino, thiomorpholino, pipéridinyle, pipérazinyle ou 2-oxo-pyrrolidinyle ;
(ii) R₂ est un groupe phényle, substitué par un groupe méthylènedioxy, éthylènedioxy, alcoxy en C₁-C₃, amino ou mono- ou di-alkyl(en C₁-C₃)amino, ou amino cyclique ;
(iii) R₃ est l'hydrogène ; et
(iv) R₄ est un groupe carboxamide de formule -CONR^{B}R^{A} dans laquelle R^{B} est l'hydrogène ou un groupe alkyle en C₁-C₃, R^{A} est un groupe alkyle en C₁-C₃, ou R^{A} et R^{B} forment conjointement -(CH₂)_{b}-, où b vaut 3, 4 ou 5.

19. Composition pharmaceutique ou vétérinaire comprenant un composé selon l'une quelconque des revendications précédentes, avec un ou plusieurs supports et/ou excipients acceptables sur le plan pharmaceutique ou vétérinaire.

20. Utilisation d'un composé selon l'une quelconque des revendications 1 à 18 dans la préparation d'une composition destinée au traitement d'une maladie virale, de maladies inflammatoires telles que l'arthrite rhumatoïde, l'asthme, la sclérose en plaques, le diabète de type I, le lupus, le psoriasis et l'affection abdominale inflammatoire ; la mucoviscidose, une maladie associée à l'angiogenèse telle que la rétinopathie diabétique, l'hémangiome, et l'endométriose ; ou à la protection des cellules normales contre une toxicité induite par une chimiothérapie ; où des maladies dans lesquelles une impossibilité de subir l'apoptose est un facteur sous-jacent ; ou à la protection contre une hypoxie-lésion ischémique due à l'élévation de Hsp90 dans le coeur et le cerveau ; la tremblante du mouton/maladie de Creutzfeldt-Jakob, la chorée de Huntington ou la maladie d'Alzheimer.

21. Utilisation d'un composé selon l'une quelconque des revendications 1 à 18 dans la préparation d'une composition destinée au traitement du cancer.
